(19) Europäisches Patentamt / European Patent Office / Office européen des brevets

(11) **EP 4 165 389 B1**

(12) **EUROPEAN PATENT SPECIFICATION**

(45) Date of publication and mention of the grant of the patent:
**19.02.2025 Bulletin 2025/08**

(21) Application number: **21714623.2**

(22) Date of filing: **17.03.2021**

(51) International Patent Classification (IPC):
*G01N 15/06* (2024.01)     *G01N 33/543* (2006.01)
*C12Q 1/6813* (2018.01)     *G01N 21/00* (2006.01)

(52) Cooperative Patent Classification (CPC):
**G01N 33/54313; G01N 15/1433; G01N 15/1434; G01N 33/54326; G01N 33/54346;** G01N 15/01; G01N 15/075; G01N 2015/1006; G01N 2015/1486

(86) International application number:
**PCT/CN2021/081214**

(87) International publication number:
**WO 2022/016887 (27.01.2022 Gazette 2022/04)**

(54) **METHODS AND APPARATUS FOR DETECTING MOLECULES**

VERFAHREN UND VORRICHTUNG ZUM NACHWEIS VON MOLEKÜLEN

PROCÉDÉS ET APPAREIL DE DÉTECTION DE MOLÉCULES

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority:  24.07.2020  CN 202010723951
17.12.2020  CN 202011494318

(43) Date of publication of application:
**19.04.2023 Bulletin 2023/16**

(73) Proprietors:
• **Bioland Laboratory**
  **Guangzhou, Guangdong 510320 (CN)**
• **Institute of Biophysics of the Chinese Academy of Sciences**
  **Beijing 100101 (CN)**

(72) Inventors:
• **JI, Wei**
  **Guangzhou, Guangdong 510320 (CN)**
• **GU, Lusheng**
  **Guangzhou, Guangdong 510320 (CN)**
• **XU, Tao**
  **Guangzhou, Guangdong 510320 (CN)**

(74) Representative: **Kailuweit & Uhlemann Patentanwälte Partnerschaft mbB Bamberger Straße 49 01187 Dresden (DE)**

(56) References cited:
**WO-A1-2019/068269**

• **AKAMA KENJI ET AL: "Multiplexed homogeneous digital immunoassay based on single-particle motion analysis", LAB ON A CHIP, vol. 20, no. 12, 22 April 2020 (2020-04-22), pages 2113 - 2121, XP055811098, ISSN: 1473-0197, DOI: 10.1039/D0LC00079E**
• **JANINE MOK ET AL: "Digital microfluidic assay for protein detection", PROCEEDINGS OF THE NATIONAL ACADEMY OF SCIENCES, vol. 111, no. 6, 21 January 2014 (2014-01-21), pages 2110 - 2115, XP055664971, ISSN: 0027-8424, DOI: 10.1073/pnas.1323998111**
• **ROLF KOOLE ET AL: "Paramagnetic Lipid-Coated Silica Nanoparticles with a Fluorescent Quantum Dot Core: A New Contrast Agent Platform for Multimodality Imaging", BIOCONJUGATE CHEMISTRY, vol. 19, no. 12, 17 December 2008 (2008-12-17), pages 2471 - 2479, XP055014206, ISSN: 1043-1802, DOI: 10.1021/bc800368x**

## Description

## Technical Field

The present invention belongs to the field of biological detection. In particular, the present invention relates to methods and use of an apparatus for detecting molecules.

## Background Art

In the market segments of in vitro diagnostic (IVD) products, immunodiagnosis and molecular diagnosis rank among the top three. The emergence of digital PCR indicates that molecular diagnosis has firstly entered the digital age, and its development and promotion are in a rapid development stage. Immunological assays based on immunological theories and principles play an important role in the prevention, diagnosis, treatment and prognosis evaluation of clinical diseases. Taking China as an example, the proportion of immunodiagnosis in IVD market was 35% (about 20 billion RMB) in 2018. Immunodiagnosis has undergone several decades of development, including radioimmunoassay (RIA), immunocolloidal gold technique, enzyme-linked immunosorbent assay (ELISA), time-resolved fluorescence immunoassay (TRFIA), etc. The current mainstream technology is chemiluminescent immunoassay (CLIA), which accounts for about 70% of the market share. However, both the acridinium ester luminescence assay of Abbott and the electrochemical luminescence assay of Roche utilize luminescence reaction, that is, quantitative analysis is realized by detecting the overall luminescence intensity of a solution, so the detection sensitivity, the dynamic range, the required sample size and the like are limited by the detection principle and methodology, and accurate and quantitative detection cannot be realized for various disease-related molecules such as nerve factors, cancer factors, immune factors, and hormones with low abundance. The digital immunodiagnosis technology can break the bottleneck of the detection sensitivity of the existing luminescent systems from the detection principle. It realizes digital quantitative detection and analysis by directly counting individual immune complex molecules and can realize trace detection with a high sensitivity and a high dynamic range, which is hopeful to become the next generation immunodiagnosis technology in place of the core position of chemiluminescence.

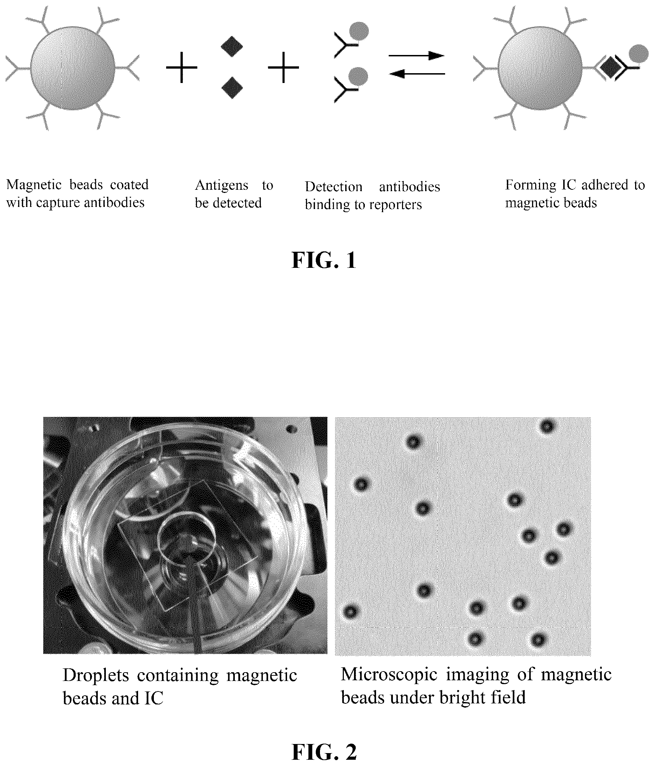

In digital immunoassay, molecule to be detected is captured by an immunolabeling method to perform fluorescent signal molecular labeling or enzyme-linked labeling, and detection of single-molecule level is realized by direct single-molecule fluorescence counting or indirect single-molecule enzymatic reaction amplification. The former requires a system with an extremely high optical detection sensitivity, and the latter needs to efficiently obtain the tiny reaction space (femtoliter-picoliter) of droplets or microwells to prevent the diffusion of fluorescent substrate generated by the reaction, thus finally achieving the readout of digital fluorescence signal. Due to the realization of the digitalization of immunoassay, the detection sensitivities of these two methods are far higher than that of the existing chemiluminescence technique platforms. Foreign enterprises and capital markets with advanced strategic vision have also started the industrial layout of digital detection technology in the immunodiagnosis market. At present, the commercialized digital detection equipments abroad mainly comprise the SiMoA system (enzyme-linked signal amplification in tiny spaces) developed by Quanterix, USA, and the SMCxPro system (high-sensitivity single-molecule detection and counting) being promoted by Merck. Both technologies use the same principle of microparticle capture and antigen enrichment as chemiluminescence methods. In the case of double antibody sandwich method, they both connect microparticles to Capture Antibody (CA), and connect antigen to CA and Detection Antibody (DA) via different epitopes, to form an Immune Complex (IC) attached to the microparticles, as shown in FIG. 1, and finally achieve the counting and concentration determination of IC in the form of optical signals by connecting DA to reporter (an enzyme or a fluorescent molecule). After the IC is formed and connected to the reporter, the two techniques achieve digital optical signal readout in different ways, wherein:

SiMoA system: a final reaction solution containing the microspheres is uniformly coated on a chip containing tens of thousands of microwells. The microspheres carrying the IC have β-galactosidase (βG), and the microwells contain the reaction substrate resorufin-β-d-galactopyranoside (RGP). RGP will produce a fluorescent substrate after an enzymatic catalysis, and the microwells will be oil-sealed to form microreactors to ensure that the fluorescent substrate will not diffuse out of single reaction microwells after the reaction, and a high concentration of the fluorescent substrate will lead to local signal amplification. The microwells containing IC microspheres will produce a locally high concentration of fluorescent substrate, and this fluorescent signal will be significantly different from the microwells without IC. The concentration of IC, i.e. the antigen to be detected, can be calculated by calculating the ratio of the number of microwells containing IC microspheres (fluorescent microwells) to all the microwells containing microspheres. The US patent No. US12/731130 protects a technical solution of this system, and discloses a method for determining the concentration of analyte molecules or particles in a fluid sample. However, the inventors have found the following drawbacks of the SiMoA system in a long-term research: (1) the cost of the instrument is too high due to the use of a self-contained instrument construction, microsphere enrichment and reaction method; (2) the solid phase support for the assay needs to be divided into spaces in advance, for example, a chip needs to be etched regularly in advance to form small wells, and the well size needs to be matched with the particle size of the microspheres, so that the preparation method of the chip is difficult and high in cost; (3) the loss of the microspheres in the detection process is excessive, and the microspheres finally entering the reaction microwells only account for 5-10% of the number of the microspheres participating in the reaction, which affects the sensitivity and the stability of the detection of a low-abundance sample; (4) the detection signal readout process needs to wait for several minutes, signal readout can be performed until the fluorescence signal of the substrate generated by the enzyme reaction reaches a certain intensity, and the time just can be tolerated for single sample detection, but the waiting time is too long for a high-throughput clinical detection, which affects the detection throughput of the system. The method is only applied to basic research at present, and it is difficult to be applied in clinic;

SMCxPro System: different from chemiluminescence and SiMoA systems, this system treats the reacted solution with a urea solution to elute the IC from the microspheres and separate them from the microspheres. At this time, the IC is also destroyed, but each fluorescent molecule in the solution will correspond to an IC, so the two are the same in quantity and concentration. The SMCxPro system utilizes a high-sensitivity optical detection system to perform random scanning detection of different positions in the solution, and performs single molecule detection and counting of the solution containing the reporter, thereby presuming the concentration of the antigen to be detected, i.e., IC. However, the inventors have found the following drawbacks of the SMCxPro system in the long-term research: (1) the instrument fittings cost is high due to the use of the high-sensitivity optical system; (2) the previous generation of instruments adopt a glass tube flow detection system, which is prone to be blocked and causes the system instability. Although the new generation of systems have solved the problem, the defect that only local sampling can be conducted can not be solved due to factors such as free molecular diffusion, and the overall concentration of the sample can only be estimated with local samples, thus influencing the sensitivity and stability of low-abundance sample detection; (3) the stability problems such as being easily bleached and quenched exist for single molecule; (4) the previous generation of instruments adopt a glass tube flow detection system, and the signal readout process of 20 microliter of final reaction solution needs more than 20 minutes. The new generation of products should be improved, but the signal detection is still based on single excitation light point excitation and photomultiplier single-point detection, and the signal readout efficiency is low. To improve the accuracy, it is necessary to traverse a large number of different positions of the solution, and the estimated detection time is also more than 1 minute, which affects the detection throughput of the system.

Chinese patent application No. 201280049085.1 discloses a biomolecule analysis method and a biomolecule analysis apparatus, which realize a wide dynamic range and rapid analysis by using biomolecule number counting in the biomolecule analysis method. The application relates to a biomolecule analysis method including the steps of: immobilizing an analyte biomolecule on the surface of a magnetic microparticle, reacting a labeled probe molecule with the analyte biomolecule, collecting and immobilizing the microparticle on a support substrate, and measuring the label on the support substrate. This application realizes the counting of the number of biomolecules by using magnetic microparticles with one molecule, and realizes rapid reaction by hybridization and reaction between the antigen and antibody in a state where the microparticles with immobilized biomolecules are dispersed. However, the inventors have found the following drawbacks existing in this application during the long-term research: (1) how to calculate the molecular concentration when more than one molecule is carried on a magnetic bead is not given, and when the proportion of the magnetic beads with the molecule exceeds 10%, there is a high probability that one magnetic bead will have two or more molecules, and then how to accurately determine the molecular concentration needs to introduce a statistical distribution model. (2) There are two errors that are easy to be introduced when the bright spot count is generated only based on the molecular fluorescence signal. First, the degrees of loss during the magnetic bead processing are different, which will affect the final number of bright spots; in addition, the pollution and impurity signals that produce bright spots are not identified and eliminated; it is necessary to increase the number of cleanings to ensure calculation accuracy. (3) The fluorescent markers such as fluorescent probes and quantum dots with weaker fluorescence signals, which are mentioned in the application, raise higher requirements for the power of light source, the magnification factor and the numerical aperture of objective lens and the sensitivity of detection camera in order to improve the signal to noise ratio (SNR) of detection, which increase the convenience and throughput of data acquisition of the system while increasing the cost.

Chinese patent application No. 20208000774.8 discloses a single molecule quantitative detection method and detection system. According to the application, in-situ signal enhanced nanoparticles with optical characteristics are utilized, molecules to be detected are labeled through chemical modification and molecular recognition technologies, so that single-molecule signals can be captured and recognized by an optical imaging equipment. The ultra-high sensitivity quantitative detection of the molecules to be detected is realized by counting the number signals of the in-situ signal enhanced nanoparticles. However, the inventors have found the following drawbacks in this application in the long-term research: (1) there are two errors that are prone to be introduced when the bright spot count or integrated intensity information is generated only based on the molecular fluorescence signal. First, the degrees of loss during the magnetic bead processing are different, which will affect the final number of bright spots; in addition, the pollution and impurity signals that also produce bright spots are not identified and eliminated, so multiple cleaning procedures are required to ensure calculation accuracy. (2) For the in-situ signal-enhanced nanoparticles mentioned in the application, too small particle size will result in weak signal which is undetectable; too large particle size will affect detection sensitivity, mainly because the immune binding force cannot support the interaction force between magnetic beads and particles. In order to balance these two extremes, the application has a strict requirement on the particle size, which is 180-480nm, but this size will still cause some antibodies with relatively weak binding capacity to be unable to effectively bind to the antigen and form immune complexes, thus affecting the detection. (3) The light source used in the application is a laser, which is of higher cost.

Akama and Noji describe the use of brightfield microscopy to quantify PSA and IL6 (Akama K, Noji H (2020) Multiplexed homogeneous digital immunoassay based on single-particle motion analysis. LAB ON A CHIP 20(1), 2113-2121). This is done with fluorescent labelled beads with capture antibodies for PSA and IL6 and the capture is done on a multiwell chip. The quantification is done among others with the help of brightfield microscopy.

Mok et al. disclose using microbeads for quantifying analytes of interest using a microfluidic platform (Mok J, Mindrinos MN, Davis RW, Javanmard M (2014) Digital microfluidic assay for protein detection. PROCEEDINGS OF THE NATIONAL ACADEMY OF SCIENCES 111(6), 2110-2115). The data is assisted by brightfield microscopy to show that the electronic detection is fast and reliable.

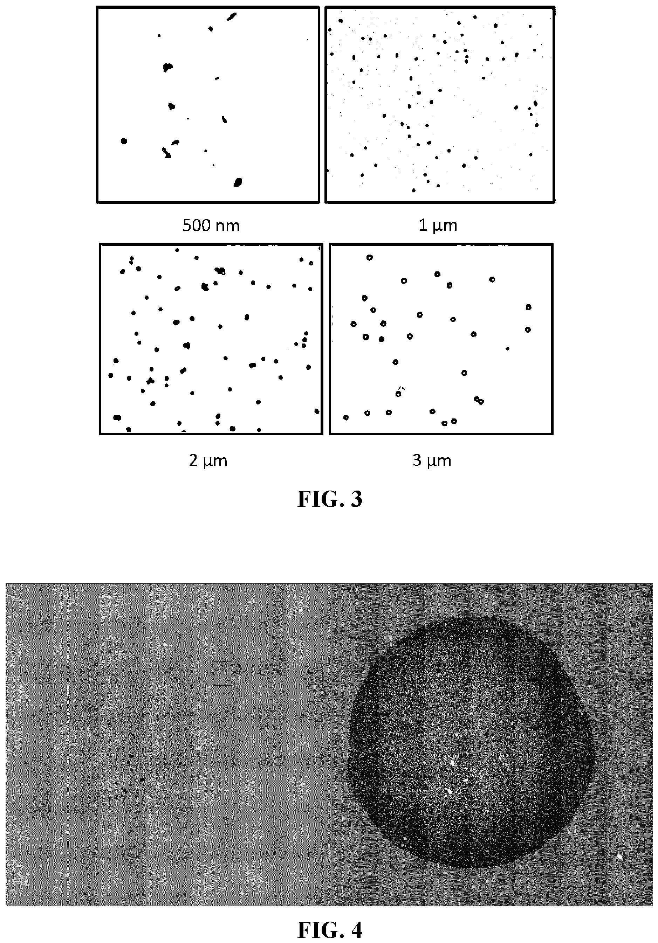

Koole et al. and WO 2019/068269 A1 describe apparatuses and computer readable media suitable for carrying out a method for detecting a signal molecule (Koole R, van Schooneveld MM, Hilhorst J, Castermans K, Cormode DP, Strijkers GJ, de Mello Donegá C, Vanmaekelbergh D, Griffioen AW, Nicolay K, Fayad ZA, Meijerink A, Mulder WJ (2008) Paramagnetic Lipid-Coated Silica Nanoparticles with a Fluorescent Quantum Dot Core: A New Contrast Agent Platform for Multimodality Imaging. BIOCONJUGATE CHEMISTRY 19(12), 2471-2479). In summary, the prior art has the following problems: (1) It is not clearly disclosed that the background signal needs to be processed, which may cause higher background signal. For example: i. the free fluorescent molecules in the solution cannot be rinsed away during the elution process of the complex captured by microparticles, and there are some unbound free fluorescent molecules in the solution; ii. there are still some impurities in the solution, and the impurities will adsorb the fluorescent molecules non-specifically. The above two aspects cause the dark field detection to be unable to distinguish the fluorescent molecules specifically bound to the magnetic beads from the free fluorescent molecules in the solution or the fluorescent molecules non-specifically bound to impurities, resulting in false positive signals (as shown in FIG. 4), thereby affecting the accuracy and sensitivity of detection; (2) magnetic beads labeled with fluorescent molecules will be lost during the processing; the lost microparticles cannot be counted when using dark field detection instead of bright field detection, which will affect the final number of microparticles containing signals, resulting in a low accuracy; (3) the magnetic beads will agglomerate to affect the accuracy of detection; (4) at present, the chip used for digital immunoassay needs to divide the spatial positions in advance, and then the captured complexes are divided into a plurality of positions spatially, so that the chip processing technology is complex, and the chip cost is high; (5) at present, digital immunoassays basically adopt a high-power lens, and the cost is high.

At present, there is an urgent need for high-sensitivity detection methods due to the requirements of novel coronavirus antigen detection, and the clinical detection of neurological factors and cancer factors, etc. However, the cost and convenience of detection in this field have affected the clinical application of single-molecule immunoassays, and there is an urgent need to develop a rapid, simple and low-cost method for effective molecular detection.

## Contents of the Invention

In view of the above problems in the prior art, the present invention has addressed the problems of complex process, low stability, low sensitivity, low accuracy and high cost in biomolecule detection, and provided methods and the use of an apparatus for detecting molecules, which utilize a common solid phase support (such as a glass slide, a multi-well plate and a flow channel) to analyse and detect biomolecules through random uniform distribution of microparticles and direct imaging under bright and dark fields, thus simplifying the detection process and improving the stability, sensitivity and accuracy, reducing cost, and facilitating promotion in multiple fields such as scientific research, clinical diagnosis, and epidemic prevention.

The above objectives of the present invention are achieved by the following technical solutions:

In a first aspect, the present invention provides a method for detecting a signal molecule, which comprises the following steps of:

(1) providing a solution comprising microparticles, wherein the microparticles comprise microparticles binding to a signal molecule to be detected;

(2) immobilizing the microparticles in the solution to the surface and/or the interior of a solid phase support;

(3) counting the microparticles in a selected field of view under a bright field;

(4) counting the microparticles binding to a signal molecule in a selected field of view under a dark field; and

(5) determining the concentration of the signal molecule according to the counting results obtained in step (3) and step (4);

wherein step (3) comprises removing the agglomerated/overlapped microparticles in the bright field, and wherein the selected field of view under the bright field and the selected field of view under the dark field are the same field of view, and counting under the bright and dark fields are performed in the same field of view.

In a second aspect, the present invention provides a method for detecting one or more signal molecules, which comprises the following steps of:

(1) providing a solution comprising microparticles, wherein a target molecule forms a complex by a specific binding reaction, the microparticles is linked to the complex, and the complex is labeled with a signal molecule;

(2) immobilizing the microparticles in the solution to the surface and/or the interior of a solid phase support;

(3) counting the microparticles in a selected field of view under a bright field;

(4) counting the microparticles binding to the complex in a selected field of view under a dark field; and

(5) determining the concentration of the signal molecule according to the counting results obtained in steps (3) and (4), and further determining the concentration of the target molecule;

wherein step (3) comprises removing the agglomerated/overlapped microparticles in the bright field, and wherein the selected field of view under the bright field and the selected field of view under the dark field are the same field of view, and counting under the bright and dark fields are performed in the same field of view.

In the method of the present invention, in step (2), the solid phase support does not need to be spatially divided to achieve a random distribution.

In the method of the present invention, the target molecule is one or more selected from the group consisting of a protein, a polypeptide, an amino acid, an antigen, an antibody, a receptor, a ligand, or a nucleic acid.

In the method of the present invention, the specific binding reaction is one or more selected from the group consisting of an immune reaction, a hybridization reaction, and a receptor-ligand interaction.

In a preferred method of the present invention, the complex may be an immune complex, and the specific binding reaction may be a sandwich or competitive immunoreaction. For example, a sandwich immunoreaction refers to that microparticles are coupled with a capture antibody to specifically bind to a first site of a target molecule so as to capture the target molecule, then a detection antibody is added to bind to a second site of the target molecule to form an immune complex, with the detection antibody being directly or indirectly labeled with a signal molecule; or a second binding site of the target molecule first binds to the detection antibody, and the conjugate then binds to the capture antibody to form microparticles of immune complex with the signal molecule, as shown in FIG. 1.

In the methods of the present invention, the microparticles may be spheres, ellipsoids, spheroids, cubes, polyhedrons, cylinders or irregular shapes. The microparticles may have a size ranging from 600 nm to 10 $\mu$m, such as 600 nm, 1 $\mu$m, 2 $\mu$m, 3 $\mu$m, 5 $\mu$m, and 10 $\mu$m; preferably from 1 $\mu$m to 5 $\mu$m.

The surface of the microparticles may be modified with one or more reactive functional groups, which may be one or more selected from the group consisting of -OH, -COOH, -NH$_2$, -CHO and -SO$_3$. In some embodiments, the capture molecule is conjugated or bound to the microparticle by physical adsorption or chemical conjugation (e.g., bridging by a bridge). The bridge may be one or more selected from the group consisting of a protein, a marker-anti-marker complex, or a crosslinker suitable for carboxyl and/or primary amine. The protein may be one or more selected from the group consisting of bovine serum albumin, ovalbumin, keyhole limpet hemocyanin, immunoglobulin, thyroglobulin, and polylysine. The crosslinking agent suitable for carboxyl and/or primary amine is one or more selected from the group consisting of dicyclohexylcarbodiimide (DCC), carbodiimide (EDC), N-hydroxybenzotriazole (HOBt) and N-hydroxysuccinimide (NHS).

In the methods of the present invention, the microparticles in the mciroparticle-containing solution may have a concentration ranging from 1 thousand to 2 million microparticles per 50 $\mu$l to 400 $\mu$l (e.g., 100 $\mu$l, 200 $\mu$l, 300 $\mu$l, preferably 10 $\mu$l to 20 $\mu$l) solution.

In the methods of the present invention, a density of individual microparticles when plated on the surface of a solid phase support is 1/cm$^2$ to 20 million/cm$^2$, such as 10/cm$^2$, 100/cm$^2$, 1000/cm$^2$, 50 million/cm$^2$, 10,000/cm$^2$ 100,000/cm$^2$, 150,000/cm$^2$, 200,000/cm$^2$, 250,000/cm$^2$, 300,000/cm$^2$, 500,000/cm$^2$, 1 million/cm$^2$, 5 million/cm$^2$, or 10 million/cm$^2$.

In the methods of the present invention, the microparticles are magnetic microparticles, preferably the microparticles are magnetic beads, which comprise a magnetic substance as a component. The magnetic substance may be a metal (an elemental metal or an alloy), a non-metal, or a complex formed by a metal and a non-metal. The metal can be, for example, iron, aluminum, nickel, cobalt, and the like; the non-metal may be, for example, a ferrite non-metal (preferably $Fe_2O_3$ or $Fe_3O_4$ magnetic nanoparticles); the complex formed by a metal and a non-metal may be, for example, a neodymium iron boron rubber magnetic composite.

Preferably, the magnetic beads are one or more selected from the group consisting of paramagnetic and superparamagnetic magnetic beads. The magnetic beads may have a diameter ranging from 600 nm to 10 $\mu$m, such as 600 nm, 1 $\mu$m, 2 $\mu$m, 3 $\mu$m, 5 $\mu$m, 10 $\mu$m; preferred from 1 $\mu$m to 5 $\mu$m.

In the methods of the present invention, the microparticles can be randomly and uniformly distributed at the surface and/or in the interior of the solid phase support, and the microparticles can still be randomly and uniformly distributed after being immobilized so as to facilitate subsequent detection.

In the methods of the present invention, the immobilized microparticles are substantially free of agglomeration or overlap with each other.

In a preferred embodiment of the present invention, the method of the present invention further comprises the steps of: enabling the microparticles (after being plated) to be uniformly distributed on the solid phase support and adsorbed to the surface of the solid phase support with a magnetic field or an electric field. For example, a magnet is placed under the solid phase support to adsorb the microparticles to the surface of the solid phase support.

In a preferred embodiment of the present invention, the method of the present invention further comprises the steps of: removing the solvent from the microparticle solution, for example, adsorbing water with a material having water-absorbing property, or naturally drying, or baking (preferably at a baking temperature of 40°C-80°C, for example, 50°C, 55°C, 60°C, 70°C).

In the methods of the present invention, the signal molecule is one or more selected from the group consisting of a chromophore, a digoxin-labeled probe, a metal nanoparticle, or an enzyme.

The chromophore is one or more selected from the group consisting of a fluorescent molecule, a quantum dot, a chemiluminescent molecule, a luminescent compound, and a dye;

The enzyme is selected from enzymes that produce a detectable signal, such as horseradish peroxidase, alkaline phosphatase, $\beta$-galactosidase, and glucose -6-phosphate dehydrogenase.

In the methods of the present invention, the signal molecule is one or more selected from the group consisting of a organic small molecule fluorescent probe, a quantum dot, a quantum dot bead, a fluorescent bead, a chemiluminescent signal molecule, a chemiluminescent molecule coated bead, a three-dimensional DNA nanostructure reporter probe, a upconversion luminescent nanomaterial bead, a rolling circle amplification fluorescent molecule amplification structure or a nucleic acid aptamer fluorescent molecule amplification structure.

Preferably, the quantum dot bead has a size less than 110 nm.

In the methods of the present invention, in step (2), the microparticles are immobilized to the surface and/or the interior of a solid phase support by an applied magnetic field and/or an electric field and/or a gel.

In the methods of the present invention, the immobilized microparticles are distributed in two dimensions on the solid phase support or distributed in three dimensions inside or on the surface of the solid phase support. When the microparticles are distributed in two dimensions on the solid phase support, the solid phase support for immobilizing the microparticles is a planar support. The microparticle solution is firstly dispersed in a gel solution and then added to the solid phase support to be solidified, so that the microparticles are distributed in three dimensions inside and/or on the surface of the solid phase support, and the solid phase support for immobilizing the microparticles may be a planar support or a well plate, etc.

In the methods of the present invention, the solid phase support is selected from the group consisting of a multi-well plate, a flat plate or a flow channel, and the solid phase support is made of silicate glass, transparent plastic or organic glass (e.g. acrylic). The solid phase support does not need special treatment, such as space division, and the detection cost is greatly reduced. When the solid phase support is a multi-well plate, the plurality of wells are used to detect a plurality of samples, and one sample is added to each well, and each sample is randomly and uniformly distributed in different wells, rather than regularly spatially dividing the multi-well plate.

In step (2), the microparticles in the solution are immobilized to the surface and/or the interior of the solid phase support, where the number of the microparticles immobilized to the surface and/or the interior of the solid phase support is uncertain, and there is no need to add a certain number of microparticles to a spatially divided region in advace.

In the methods of the present invention, the solid phase support is a multi-well plate, such as a 48-well plate, a 96-well plate, a 384-well plate, a 512-well plate, a 1024-well plate, or a 1536-well plate.

In the present invention, the solid phase support is at least partially optically transparent or substantially allows visible light to pass through. The optically transparent is meant that the transmittance of visible light is greater than or equal to 10%, or greater than or equal to 20%, or greater than or equal to 30%, 40%, 50%, 60%, 70%, 80%, or 90%.

In the methods of the present invention, the coordinates of the microparticles in the image are determined by bright field microscopic imaging.

In the methods of the present invention, the boundaries of the individual microparticles are determined in the bright field mode, e.g. by bright field microscopic imaging.

In the methods of the present invention, the coordinates of the microparticles are determined from the difference in brightness of the microparticles. In the bright field mode, the center of the microparticles is bright and the periphery is dark, and visible bright spots are formed in the center of the magnetic beads, as shown in FIG. 2, to realize the confirmation and counting of the magnetic beads.

In a preferred embodiment of the present invention, the difference in brightness of the microparticles is the brightness difference of the microparticles themselves.

In a preferred embodiment of the present invention, the agglomeration/overlap of the microparticles is determined in the bright field mode, only individual microparticles are counted as a basis for calculating the concentration of the molecules to be detected by removing the agglomerated/overlapped microparticles.

In the methods of the present invention, the counting in step (4) is determined by the coordinates of the microparticles in the image.

In the methods of the present invention, the counting under the bright and dark fields is usually performed in the same field of view.

The accuracy and sensitivity of the detection can be improved by imaging in a bright field and a dark field, respectively; if only the dark field detection is performed, it is impossible to distinguish the fluorescent molecules specifically bound to the magnetic beads from the free fluorescent molecules in the solution or the fluorescent molecules non-specifically bound to impurities, which are prone to produce false positive signals. For example, in the rectangular area in Figure 4, there are no magnetic beads in the bright field, but signals can be detected in the dark field.

In the methods of the present invention, counting and statistics of microparticles and/or signal molecules are performed after microscopic imaging.

Preferably, the methods of the present invention further comprise counting the number of microparticles in the bright field view and the number of microparticles comprising a signal molecule in the corresponding dark field, thereby calculating the concentration of the target molecule to be detected. Calculation methods include, but are not limited to: determining the concentration of the molecule to be detected according to the proportional relationship between the number of the microparticles comprising the signal molecule and the number of the microparticles in a bright field view, in combination with a test curve obtained by standard substance with different concentrations; and determining the concentration of the molecule to be detected according to the average signal intensity on the microparticles comprising the signal molecule, in combination with a test curve obtained by standard substance with different concentrations.

In the method of the present invention, there may be one or more target molecules, and the microparticle comprises at least one capture molecule, for example, when there are two target molecules, the microparticles:

are at least partially coupled with two capture molecules; or
are at least partially immobilized with a first capture molecule, and at least partially immobilized with a second capture molecule.

The capture molecules specifically bind to the first binding sites of the corresponding target molecules respectively, detection molecules labeled with signal molecules are added and specifically bind to the second binding sites of the target molecules respectively, and different target molecules are determined through different signal molecules labeled by the detection molecules or shapes of microparticles.

The methods of the present invention may be used for a non-diagnostic purpose.

In a third aspect, the present invention provides use of the above methods in the preparation of a diagnostic reagent for the detection of a biomolecule.

In the use of the present invention, the diagnostic reagent is a detection reagent for a protein or a nucleic acid, and the reagent comprises a microparticle, a capture molecule, a detection molecule and a signal molecule; optionally, the diagnostic reagent further comprises a buffer reagent, a coupling reagent and a cleaning reagent; optionally, the diagnostic reagent may further comprise a detection means.

In a fourth aspect, the present invention also provides the use of a detection apparatus for implementing the above methods, which comprises:

a solid phase support capable of immobilizing microparticles, the microparticles comprising microparticles binding to a signal molecule to be detected and being dispersed at the surface and/or in the interior of the solid phase support;
at least one first light source irradiating microparticles within a selected field of view of the solid phase support to form bright field signals related to the total number of microparticles, and at least one second light source irradiating microparticles within the selected field of view of the solid phase support to form dark field signals related to the total number of microparticles binding to a signal molecule to be detected;
a signal acquisition unit for acquiring bright field signals and dark field signals; and
a signal processing unit for determining the concentration of the signal molecule according to the acquired bright field signals and dark field signals.

In the use of the apparatus of the present invention, the solid phase support is disposed above or below the signal acquisition unit, and the solid phase support is configured to be removable from above or below the signal acquisition unit.

In the use of the apparatus of the present invention, the solid phase support may comprise at least one flow channel with an inlet and an outlet, and a solution comprising the microparticles is dispersed in the flow channel. Preferably, the solid phase support comprises a flow channel for feeding and discharging the solution comprising the microparticles, wherein at least a portion of the flow channel overlaps an optical path of the light detection unit to allow detection using the first light source and the second light source. Preferably, the flow channel is selected from a glass tube or a microfluidic chip.

In the use of the apparatus of the present invention, the solid phase support may also be a multi-well plate or a flat plate.

In the use of the apparatus of the present invention, the apparatus further comprises a magnetic field generating device or an electric field generating device for immobilizing and dispersing the microparticles at the surface and/or in the interior of the solid phase support. Preferably, the magnetic force generating device may preferably be a magnet.

In the use of an apparatus of the present invention, the solid phase support is preferably a turntable which can rotate relative to the signal acquisition unit, wherein the turntable comprises at least one optically transparent detection site, e.g. a blind hole, which is detected by the signal acquisition unit when the detection site is located in an optical path of the signal acquisition unit. The number of blind holes may be 1 or more, for example 2, 3, 4, 5, 6, 7, 8, 9, 10, 11, or 12. Preferably, the turntable is configured to rotate sequentially between a plurality of stations in a stepwise manner, and to perform the following operations on a solution to be detected at the detection site at the plurality of stations: immobilizing and rinsing microparticles in the solution. Preferably, the plurality of stations comprise a detection station located in an optical path of the signal acquisition unit, at least one pre-treatment station located upstream of the detection station, and at least one post-treatment station located downstream of the detection station, wherein an operation of immobilizing and dispersing microparticles in the solution is performed at the pre-treatment station, and a rinsing operation is performed at the post-treatment station.

The turntable is preferably a disk or an annular disk. "Annular disk" refers to a ring-shaped disk, and a typical annular disk refers to a remaining part of a large disk from which a small concentric disk is excavated. In the present invention, the annular disk does not need to be completely closed, it may have one or several notches, and the appearance of the ring is not limited to a circle, it may be an irregular shape, such as a polygon, preferably a regular polygon.

According to the description of the present invention, corresponding devices required for forming a signal acquisition unit and a signal processing unit would be readily conceivable to a person skilled in the art. For example, the signal acquisition unit comprises an amplification assembly, a lens, an optical filter, and a photographing assembly, etc.; and the signal processing unit comprises a computer that controls image acquisition and storage. Preferably, the amplification assembly is adapted to amplify microparticles within the selected field of view. Preferably, the amplification assembly is an objective lens; more preferably, the objective lens is a low power objective lens, such as 4X, 10X, 20X, 40X. Compared with a high-power objective lens, a low-power objective lens can detect multiple samples/droplets in one field of view, which improves detection efficiency and reduces detection cost.

In the use of the apparatus of the present invention, the apparatus further comprises a displacement mechanism for actuating the signal acquisition unit and/or the solid phase support.

In the use of the apparatus of the present invention, preferably, the displacement mechanism is one or more selected from the group consisting of a one-dimensional displacement stage, a two-dimensional displacement stage, and a three-dimensional displacement stage.

In the use of the apparatus of the present invention, preferably, the displacement mechanism is a two-dimensional displacement stage to allow bright field and dark field microscopic imaging of individual microparticles to be acquired in a planar motion manner while the individual microparticles are dispersed on the surface of the solid phase support.

In the use of the apparatus of the present invention, preferably, the displacement mechanism is a three-dimensional displacement stage to allow bright field and dark field microscopic imaging of individual microparticles to be acquired layer by layer in a spatial motion manner while the individual microparticles are dispersed inside the solid phase support.

In embodiments, the apparatus comprises a computer-readable storage medium for storing programs for executing the methods described in the first aspect and the second aspect, and/or data generated by executing the methods.

The computer storage medium is configured to store a computer instruction, a program, a code set, or a instruction set that, when executed on a computer, cause the computer to perform the method of detecting a signal molecule on microparticles as described above, the method of analyzing a target molecule as described above, or the method of determining multiple types of target molecules as described above.

Any combination of one or more computer-readable media may be employed. The computer-readable medium may be a computer-readable signal medium or a computer-readable storage medium. The computer-readable storage medium includes, but is not limited to an electrical, magnetic, optical, electromagnetic, infrared, or semiconductor system, apparatus, or device, or any combination thereof. More specific examples of the computer-readable storage medium include, but are not limited to: an electrical connection having one or more wires, a portable computer disk, a hard disk, a random access memory (RAM), a read-only memory (ROM), an erasable programmable read-only memory (EPROM or a flash memory), an optical fiber, a portable compact disk read-only memory, an optical storage device, a magnetic storage device, or any suitable combination thereof. In this specification, the computer-readable storage medium may be any tangible medium that contains or stores a program for use by or in combination with an instruction execution system, apparatus, or device.

The computer-readable signal medium may comprise a data signal that is propagated in a baseband or as a part of a carrier wave, in which a computer-readable program code is carried. Such a propagated signal may take a variety of forms, including but not limited to, an electromagnetic signal, an optical signal, or any suitable combination thereof. The computer-readable signal medium may also be any computer-readable medium other than the computer-readable storage medium, and the computer-readable medium can send, propagate, or transmitt a program for use by or in combination with an instruction execution system, apparatus, or device.

Program code contained in the computer-readable medium may be transmitted using any appropriate medium, including but not limited to a wireless connection, a electrical wire, a optical cable, a RF, or any suitable combination thereof.

The computer program code for performing operations of the present invention may be compiled by using one or more programming languages or a combination thereof. The programming languages include object-oriented programming languages such as Java, Smalltalk, C++, as well as conventional procedural programming languages such as C language or similar programming languages. The program code may be executed entirely on a user's computer, partly on a user's computer, as a stand-alone software package, partly on a user's computer and partly on a remote computer, or entirely on a remote computer or server. In the case of a remote computer, the remote computer may be connected to a user's computer through any type of network, including a local area network (LAN) or a wide area network (WAN). Alternatively, it may be connected to an external computer (for example, using an Internet service provider to connect through the Internet).

In embodiments, the apparatus comprises an electronic device comprising the computer-readable storage medium.

The electronic device comprises one or more processors; and

> a memory device for storing one or more programs, wherein
> when the one or more programs are executed by the one or more processors, the one or more processors implement the method of detecting a signal molecule on microparticles as described above, the method of analyzing a target molecule as described above, or the method of analyzing a plurality of types of target molecules as described above (which may be any one or more steps therein).

Alternatively, the electronic device may further comprise a transceiver. The processor and the transceiver are connected, for example, through a bus. It should be noted that the number of the transceiver is not limited to one in practical applications, and the structure of the electronic device is not to be construed as limiting the embodiments of the present application.

The processor may be a CPU, a general-purpose processor, a DSP, a ASIC, a FPGA or other programmable logic device, a transistor logic device, a hardware component, or any combination thereof. The processor may implement or execute various examplary logical block diagrams, modules, and circuits that are described in connection with the disclosure of this application. The processor may alternatively be a combination for implementing a computing function, for example, a combination comprising one or more microprocessors and a combination of DSP and microprocessor.

The bus may comprise a path for transmitting information between the above assemblies. The bus may be a PCI bus, an EISA bus, or the like. The bus may be classified into an address bus, a data bus, a control bus, or the like. The memory may include but not limited to: a ROM or other type of static storage devices that can store static information and instructions, a RAM or other type of dynamic storage devices that can store information and instructions; or an EEPROM, a CD-ROM or other optical disk storages, an optical disc storage (including a compact optical disc, a laser disc, an optical disc, a digital versatile disc, a Blu-ray disc, or the like), a magnetic disk storage medium or other magnetic storage devices, or any other medium that can be configured to carry or store desired program code in a form of an instruction or a data structure and that is accessible to a computer.

In the electronic device, preferably, the mentioned computer instruction, program, code set or instruction set can have any one or more functions selected from the following:

> (1) calculating the number of the microparticles;
> (2) adjusting the light intensity of the bright field to obtain a higher signal-to-noise ratio;
> (3) when the signal molecule is a fluorescent substance, adjusting the excitation light wavelength and/or intensity to obtain signals, and/or obtaining a higher signal-to-noise ratio;
> (4) adjusting the focal length of the photographing assembly, and/or adjusting the distance between the photographing assembly and the solid phase support, and/or switching the photographing field of view, and/or adjusting the exposure time;

(5) performing image recognition on the microscope image data of microparticles under the bright field, extracting the image position information confirmed as individual microparticles, and comparing it with the image position information of the signal molecule to determine the ratio of x to y and/or z;

(6) converting the obtained numerical value by using a standard curve obtained by a standard substance to obtain concentration information of the target molecule;

(7) establishing a signal molecule intensity threshold value for the microparticles, and identifying the microparticles with an intensity lower than the threshold value; and/or eliminating the interference of impurities and spontaneous signal molecules (such as autofluorescence) in the sample;

(8) confirming the boundary of the microparticles to identify the microparticles which are in contact, agglomerated or overlapped with each other; and

(9) eliminating the agglomerated or overlapped microparticles from the population.

In the electronic device, preferably, the mentioned computer instruction, program, code set or instruction set is executed so that, when the microparticles are distributed in two dimensions and immobilized on the solid phase support, the system acquires bright field and dark field signals of all the microparticles distributed in two dimensions by moving a lateral displacement stage; when the microparticles are distributed in three dimensions and immobilized on the solid phase support, the system acquires bright field and dark field signals of the microparticles at each layer in the three-dimensional space by moving a axial one-dimensional displacement stage or a three-dimensional displacement stage.

**Terms**

In the context of the present invention, the term "immobilization" refers to a state in which the relative position of the microparticles at the surface/in the interior of the solid phase support (e.g., the relative position between the magnetic beads and/or the relative position between the magnetic beads and the solid phase support) is substantially unchanged, in order to facilitate counting of the number of signal molecule on the magnetic beads and/or the number of the magnetic beads, when the microparticles and the solid phase support exist simultaneously. In a preferred embodiment of the present invention, the solid phase support can make this immobilization more robust by interaction of the molecules on the solid phase support with molecules modified on the surface of the magnetic beads. The mode of interaction may be a covalent bond, a ionic bond, Van der Waals force, a hydrogen bond, gravity, magnetic force, or any combinations thereof. In some embodiments, the mode of interaction between the microparticles and the solid phase support is achieved by bridging as described above.

In the context of the present invention, the terms "substantial", "substantially" and variant thereof are intended to indicate that the feature being described is equal to or approximately equal to the stated value or description. For example, a "substantially flat" surface is intended to mean a flat or nearly flat surface. Further, as defined above, "substantially similar" is intended to mean that two values are equal or approximately equal. In some embodiments, "substantially similar" may represent values that differ from each other by within about 10%, such as within about 5% from each other or within about 2% from each other.

In the context of the present invention, the term "quantum dot bead" refer to nanoparticle that encapsulates a plurality of quantum dots.

In the context of the present invention, the term "fluorescent bead" refer to nanoparticle that encapsulates a plurality of fluorescent molecules.

In the context of the present invention, the term "dark field" refers to an environment that facilitates the detection of a signal molecule. A typical form of detection comprises photographing and collecting the optical signals of signal molecule, and alternatively comprises determining the presence or absence of the signal molecule and calculating the signal intensity of signal molecule. The above photographing methods may include, but are not limited to, scattered light imaging using dense particles, up-conversion luminescence imaging, chemiluminescence signal molecule imaging, and fluorescence imaging. In a preferred embodiment of the present invention, the dark field is an environment substantially free of visible light, providing an environment with a higher signal-to-noise ratio for signal molecule detection. For example, when the signal molecule is a fluorescent signal, the dark field can be an environment substantially free of visible light, but with excitation light and the like, so as to facilitate the photographing of the fluorescent signals and improve the image signal-to-noise ratio.

Counting of microparticles and/or signal molecules

In a preferred embodiment of the present invention, microparticles and microparticles comprising a signal molecule are counted under bright field and dark field mode microscopes, respectively.

In a particular field of view, the number of microparticles in the bright field is represented by x, and the number of microparticles comprising a signal molecule in the dark field is represented by y.

In a preferred embodiment of the present invention, the methods of the present invention further comprise counting the sum of the signal intensity on the microparticles with a signal molecule in the dark field mode (the sum of the signal intensity is represented by z).

In a preferred embodiment of the present invention, the statistical counting of microparticles and/or signal molecules are performed by microscopic imaging.

In a preferred embodiment of the present invention, the number x of microparticles is determined by electromagnetic wave imaging or sonic imaging technique.

Electromagnetic waves transmit energy and momentum in the form of wave in space by electric field and magnetic field that oscillate in phase and are perpendicular to each other, and the propagation direction is perpendicular to the plane formed by the electric and magnetic fields. The electromagnetic wave may be one or more selected from the group consisting of radio wave, microwave, infrared ray, visible ray, ultraviolet ray, X-ray, and gamma ray. Preferably, the electromagnetic wave is visible light having a wavelength between about 380 nm and 780 nm. Preferably, the sound wave is ultrasonic wave.

In a preferred embodiment of the present invention, the number x of microparticles in a particular field of view is counted by microscopic bright field imaging. The acquisition of the number y of microparticles with a signal molecule and the signal intensity sum z in a particular field of view are determined according to the type of signal molecule. In a preferred embodiment of the present invention, the number y of microparticles with a signal molecule is determined by dark field imaging of the signal molecule.

In a preferred embodiment of the present invention, the number of the particular fields is n, the number $x_i$ of the microparticles in each field and the number $y_i$ of the microparticles with a signal molecule and/or the signal intensity sum $z_i$ are counted, and the values of x, y and/or z are calculated, respectively; where n and i are non-zero natural numbers,

$$x = \sum_{i=1}^{n} x_i, \quad y = \sum_{i=1}^{n} y_i,$$

$$z = \sum_{i=1}^{n} z_i.$$

The signal molecule concentration information can be obtained by calculating the three values of x, y, and z in combination with a concentration curve of standard substance, including the proportional relationship, which can be the proportional relationship between x and y, between x and z, between y and z, or between x, y and z, or the parameters obtained after they are multiplied by a specific coefficient or transformed, which do not affect the calculation of the signal molecule intensity by those skilled in the art. For example, when y/x=1, it means that all microparticles carry a signal molecule; when y/x=0, it means that no microparticles carry a signal molecule, this ratio is preferably used to calculate the number of signal molecule together with the detection curve for standard substance with different concentrations.

In a preferred embodiment of the present invention, when the ratio of y to x is greater than 10% (e.g., 20%, 30%, 40%, 50% or more), the method of determining the number of signal molecule comprises using the average intensity of the signal molecule in combination with a detection curve for standard substance with different concentrations.

In a preferred embodiment of the present invention, during the counting of microparticles and/or signal molecule, when calculating any one or more of x, y, and z (for example, x, y; x, z; y, z), no agglomerated/overlapping microparticles are counted.

In a preferred embodiment of the present invention, alternatively, the sample may be processed in parallel using a multi-well plate to improve the detection throughput.

Target molecule

In a preferred embodiment of the present invention, the target molecule is a cell, an organelle, a microorganism, a nucleic acid, a protein, a polypeptide or a small molecule compound with a molecular weight of less than 1000, preferably a protein, a polypeptide, or a nucleic acid.

Common low abundance detection substances in the prior art include, but are not limited to neurofactors such as TNFα, IFN-α, and IFN-y, inflammatory factors such as IL-1α, IL-1β, IL-6, and IL-13; and cancer factors such as PSA, CEA, AFP, and CA19-9.

In a preferred embodiment of the present invention, the microorganism comprises a virus, a bacterium, and a fungal cell.

In a preferred embodiment of the present invention, the virus is one or more selected from the group consisting of adenoviridae, arenaviridae, astroviridae, bunyaviridae, caliciviridae, flaviviridae, hepeviridae, mononegavirales, nidovirales, picornaviridae, orthomyxoviridae, papillomaviridae, parvoviridae, polyomaviridae, poxviridae, reoviridae, retroviridae, and togaviridae.

In a preferred embodiment of the present invention, the bacterium is one or more selected from the group consisting of Staphylococcus, Streptococcus, Listeria, Erysipelothrix, Renibacterium, Bacillus, Clostridium, Mycobacterium, Actinomycetes, Nocardia, Corynebacterium, and Rhodococcus, and/or one or more selected from the group consisting of Bacillus anthracis, Erysipelothrix rhusiopathiae, Clostridium tetani, Listeria monocytogenes, Clostridium chauvoei, Mycobacterium tuberculosis, Escherichia coli, Proteusbacillus vulgaris, Shigella dysenteriae, Bacillus pneumoniae, Bacterium burgeri, Clostridium perfringen, Haemophilus influenzae, Haemophilus parainfluenzae, Moraxella catarrhalis, Acinetobacter, Yersinia, Legionella pneumophila, Bordetella pertussis, Bordetella parapertussis, Shigella, Pasteurella, Vibrio cholerae, and Vibrio Parahemolyticus.

In a preferred embodiment of the present invention, the fungus is one or more selected from the group consisting of Coccidioides immitis, Blastomyces coccidioides, Histoplasma capsulatum, Histoplasmosis duboisii, Blastomyces loboi, Paracoccidiodes brasiliensis, Blastomyces dermatitidis, Sporothrix schenckii, Penicillium marneffei, Candida albicans, Candida glabrata, Candida tropicalis, Candida lusitaniae, Aspergillus, Exophiala jeanselmei, Fonsecaea pedrosoi, Fonsecaea compacta, Phialophora verrucosa, Exophiala dermatitidis, Geotrichum candidum, Pseudallescheria boydii, Cryptococcus neoformans, Trichosporon cutaneum, Rhizopus oryzae, Mucor indicus, Absidia corymbifera, Syncephalastrum racemosum, Basidiobolus ranarum, Conidiobolus coronatus, Conidiobolus incongruus, Rhinosporidium seeberi, Hyalohyphomycetes and Dematiaceous hyphomycetes.

In the context of the present invention, the term "nucleic acid" refers to any molecule comprising a nucleic acid, including but not limited to DNA or RNA. The term encompasses a sequence of DNA or RNA comprising any known base analog, including but not limited to: 4-acetylcytosine, 8-hydroxy-N6-methyladenosine, aziridinylcytosine, pseudoisocytosine, 5-(carboxyhydroxymethyl)uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, $\beta$-D-mannosylnucleoside Q, 5'-methoxycarbonylmethyluracil, 5-methoxyuracil, 2-methylthio-N6-isopentenyladenine, uracil-5-oxymethyl acetate, uracil-5-oxyacetic acid, oxybutoxysine, 5-methyl-2-thiouracil, 2-thiouracil, 4-thiouracil, 5-methyluracil, N-uracil-5-oxymethyl acetate, uracil-5-oxyacetic acid, pseudouracil, nucleoside Q, 2-thiocytosine, and 2,6-diaminopurine.

In a preferred embodiment of the present invention, the nucleic acid is miRNA or shRNA.

In the context of the present invention, the term "polypeptide" refers to a molecule comprising at least two amino acid residues linked by peptide bonds to form a polypeptide. The term "polypeptide" also comprises a domain of protein. A small polypeptide of less than 50 amino acids may be referred to as a "peptide". Preferrably, the polypeptide is a disease marker, such as a tumor marker.

In a preferred embodiment of the present invention, the small molecule compound is a small molecule compound having a molecular weight of below 1000 (or below 500).

In a preferred embodiment of the present invention, the binding modes of capture molecule-detection molecule and capture molecule-target molecule are each independently selected from the group consisting of: biotin or a derivative thereof/streptavidin, biotin or a derivative thereof/avidin, biotin or a derivative thereof/NeutrAvidin, biotin or a derivative thereof/antibody against biotin or a derivative, hapten/antibody, antigen/antibody, polypeptide/antibody, receptor/ligand, digoxin/digoxin ligand, carbohydrate/lectin, polynucleotide/complementary polynucleotide, and nucleic acid aptamer/identifier for nucleic acid aptamer; wherein the derivative of biotin is any one of D-biotin, activated biotin, biocytin, ethylenediamine biotin, cadaverine biotin, and desthiobiotin.

Among the above-mentioned binding modes, only the way of binding is limited, but no limitation is made on the state in which the capture molecules, the detection molecules, and the target molecules bind to each other or the binding details thereof. Taking the antigen/antibody binding mode as an example, the capture molecule may be an antibody, and the target molecule may be an antigen; or the capture molecule may be an antigen, and the target molecule may be an antibody; or the capture molecule may be a mixture conjugated to or comprising an antibody, and the target molecule may be a mixture conjugated to or comprising antigens or antibodies; alternatively, the capture molecule and the target molecule may carry an additional fusion protein or a label.

The capture/detection antibody is classified according to antibody specificity characteristics, and can be one or more of a polyclonal antibody, a monoclonal antibody, a single chain antibody, an antigen-binding fragment, and a nanoantibody. The capture antibody is classified according to the source, and may be one or more of a murine-derived antibody, a rabbit-derived antibody, an sheep-derived antibody, and an alpaca-derived antibody.

In a preferred embodiment of the present invention, the signal molecule may be pre-labeled with the detection molecule, and then bind to the target molecule or capture molecule. The signal molecule can also bind to the target molecule or the capture molecule, and then is labeled by the detection molecule.

In a preferred embodiment of the present invention, the concentration of the target molecule is calculated based on the detection result of the signal molecule.

The method of the present invention is also suitable for detecting various types of target molecules. The species of the capture molecule may be 1, 2, 3, 4, 5, 6, 7, 8, 9, 10 or more. Accordingly, the types of target molecule and the detection molecule should be less than or equal to that of the capture molecule.

In a preferred embodiment of the present invention, each of the plurality of detection molecules carries a distinguishable and different signal molecule in a manner including, but not limited to, different colors, different fluorescences, and different molecular weights.

Due to the large number of microparticles, when it is desired to detect target molecule A', the corresponding capture molecule A can be dispersed and coated on the surface of different microparticles. For example, a part of microparticles can be immobilized with the capture molecule $a_1$, another part of microparticles can be immobilized with the capture molecule $a_2$, yet another part of microparticles can be immobilized with the capture molecule $a_3$, and $a_1 \cup a_2 \cup a_3 = A$; or one microparticle comprises one type of capture molecules, which can be the same or different; or one microparticle captures two or more different types capture molecules; or a part of microparticles capture one type of capture molecules, and the other part of microparticles capture a plurality of types of capture molecules, and the like.

In a preferred embodiment of the present invention, a plurality of types of detection molecules may carry one type of signal molecules, and different target molecules to be detected can be distinguished by the shapes of microparticles, which can be a sphere, a spheroid, a cube, a polyhedron or an irregular shape. The microparticles with different shapes can be coupled with different capture antibodies, thus specifically binding to different target molecules to realize the detection of the plurality of types of target molecules.

It should be noted that the present invention does not specifically limit the number of capture molecules immobilized on a unit microparticle. Even if there is only one type of capture molecules, the number of capture molecules can be one or more.

## Brief Description of the Drawings

The embodiments of the present invention will be described in detail in connection with the drawings, in which:

FIG. 1 is a schematic representation of a magnetic bead which is coated with capture antibodies to capture antigens and forms an immune complex on the surface thereof according to one embodiment of the present invention;

FIG. 2 is a photograph of magnetic beads taken under a bright field microscopic imaging mode according to one embodiment of the present invention;

FIG. 3 is a photograph of a solution of plated magnetic beads having different particle sizes taken in a bright field microscopic imaging mode according to one embodiment of the present invention;

FIG. 4 is a photograph of a solution of plated magnetic beads separately taken in bright field and dark field microscopic imaging modes according to one embodiment of the present invention.

FIG. 5 shows the detection result of different concentrations of Biotin-Qbeads with streptavidin-modified magnetic beads according to one embodiment of the present invention;

FIG. 6 shows the detection result of expressed and purified Spike protein according to one embodiment of the present invention;

FIG. 7 shows the detection result of pseudovirus expressing a novel coronavirus S protein on the surface according to one embodiment of the present invention;

FIG. 8 shows the detection result of IL-6 according to one embodiment of the present invention;

FIG. 9 is a schematic representation of the turntable according to one embodiment of the present invention;

FIG. 10 is a schematic representation of the apparatus for detecting molecules according to one embodiment of the present invention; and

FIG. 11 is a schematic representation of the position of the turntable relative to the objective lens according to one embodiment of the present invention;

whrerein 1-position 1; 2-position 2; 3-position 3; 4-position 4; 5-position 5; 6-position 6; 7-position 7; 8-position 8; 201-turntable; 202-blind hole; 203- rotary shaft; 301-magnetic bead solution; 302-blind hole containing a solution of magnetic beads to be detected; 303-bright field light source; 304-condenser lens; 305-objective lens; 306-fluorescent light source; 307-lens; 308-dichroic beamsplitter; 309-filter; 310-lens; 311-camera.

## Best Modes **for Carrying Out the Invention**

The present invention will be further described in detail below in connection with the specific examples. The examples given are only for the purpose of illustrating the present invention, but not intended to limit the scope of the present invention.

## Use of the Detection Apparatus

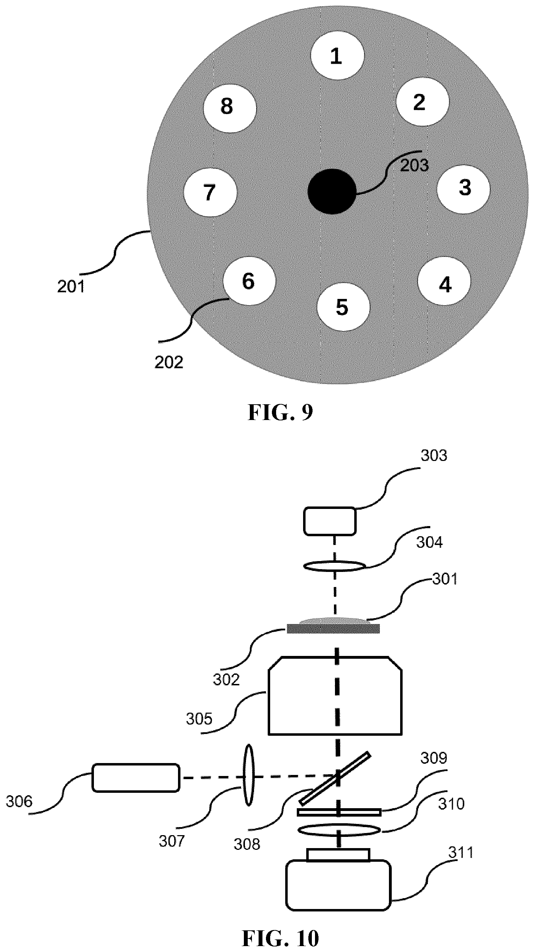

Referring to FIG. 9 to FIG. 11, the apparatus for detecting a target molecule comprises:

a solid phase support 201 capable of immobilizing the microparticles in a microparticle solution 301, wherein the microparticles comprise the microparticles binding to a signal molecule to be detected, and are dispersed at the surface and/or in the interior of the solid phase support;

at least one first light source 303 and at least one second light source 306, wherein the microparticles in a selected field of view on the solid phase support 201 (such as the magnetic beads in a blind hole 302 of a turntable 201 containing a solution of magnetic beads to be detected) are irradiated with the light emitted by the first light source 303 through a condenser lens 304, so as to form bright field signals related to the total number of the microparticles; the microparticles in a selected field of view on the solid phase support 201 are irradiated with the fluorescence emitted by the second light source 306 sequentially through a lens 307 and a dichroic beamsplitter 308 to form dark field signals related to the total number of microparticles binding to the signal molecule to be detected; wherein the first light source 303 and the second light source 306 may be provided by LEDs;

a signal acquisition unit for acquiring the bright field signals and dark field signals, respectively; the signal acquisition unit comprises an amplification assembly objective lens 305, a lens 310, a filter 309 and a photographing assembly camera 311, and the bright field signals and the dark field signals reach the camera 311 through the objective lens 305, the lens 310 and the filter 309 in sequence for microscopic imaging; and

a signal processing unit (not shown) for determining the concentration of the target molecule according to the acquired bright field signals and dark field signals.

In the use of the apparatus for detecting a target molecule of the present invention, the solid phase support is a turntable 201 capable of rotating with respect to the signal acquisition unit as shown in FIG. 9 or FIG. 10. Referring to FIG. 10, a silicate glass turntable 201 as a solid phase support is disposed above the signal acquisition unit and can be removed from above the signal acquisition unit. In other alternative embodiments, the solid phase support is a flat plate, such as a glass slide, and the microparticles in the solution 301 are randomly distributed evenly over the surface of the flat plate. In other alternative embodiments, the solid phase support has at least one flow channel comprising an inlet and an outlet. The microparticles in the solution 301 are uniformly distributed in the flow channel. At least a part of the flow channel overlaps an optical path of the light detecting unit to allow detection by the first light source 303 and the second light source 306.

The material of the turntable 201 may be quartz or glass. The turntable 201 comprises at least one optically transparent detection site which is detected by the signal acquisition unit when the detection site is located in an optical path of the signal acquisition unit. As shown in FIG. 9, the turntable 201 has eight blind holes 202 as the detection sites, and one of the blind holes 302 is a blind hole for containing a solution of the microparticles to be detected.

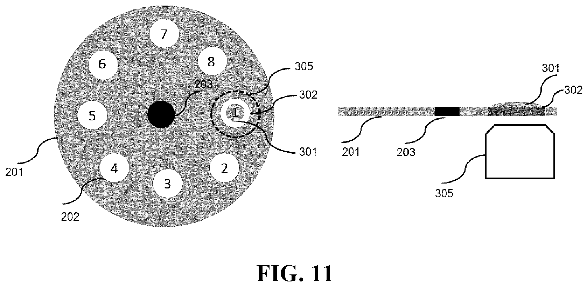

The turntable 201 as a solid phase support can rotate in a plane above the signal acquisition unit. FIG. 11 shows that the blind hole 302 containing a solution of the microparticles to be detected is positioned above the objective lens 305 by rotation. The turntable 201 is configured to rotate sequentially between a plurality of stations in a stepwise manner and to perform the following operations on a solution to be detected at the detection site at the plurality of stations: immobilizing, dispersing and rinsing the microparticles (e.g. magnetic beads) in the solution. The plurality of stations comprise a detection station located in an optical path of the signal acquisition unit, at least one pre-treatment station located upstream of the detection station, and at least one post-treatment station located downstream of the detection station, wherein an operation of immobilizing and dispersing microparticles in the solution is performed at the pre-treatment station, and a rinsing operation is performed at the post-treatment station.

The apparatus for detecting molecules further comprises a magnetic field generating device or an electric field generating device (not shown) for immobilizing and dispersing the microparticles at the surface and/or in the interior of the solid phase support.

In a preferred embodiment, the microparticles are magnetic beads which are one or more selected from the group consisting of paramagnetic beads and superparamagnetic beads. The magnetic beads have a particle size ranging from 600 nm to 10 μm.

The apparatus further comprises a displacement mechanism (not shown) for actuating the signal acquisition unit and/or the solid phase support, wherein the displacement mechanism is preferably one or more selected from the group consisting of a one-dimensional displacement stage, a two-dimensional displacement stage, and a three-dimensional displacement stage; for example, the displacement mechanism may be a two-dimensional displacement stage to allow bright field and dark field microscopic imaging of individual microparticles to be acquired in a planar motion manner while the individual microparticles are dispersed on the surface of the solid phase support.

Referring to FIG. 11, the reacted microparticles solution 301 with the immunocomplex of signal molecule is pipetted into the blind holes 202 of the transparent solid phase support, which at this time corresponds to position 5 of the eight blind holes 202 on the turntable 201 in FIG. 11. The rotary table drives the rotary shaft 203 to sequentially move the sample from position 5 to positions 4, 3, 2 and 1 to perform the steps of adding liquid agar, controlling the spatial distribution of the microparticles by external force, cooling the solidable agar to immobilize the microparticles, and acquiring signals, and the steps at positions 2 and 4 may be omitted. At position 1, the first light source 303 (i.e. a bright field light source) generates a bright field image of the magnetic beads through a condenser lens 304 and a blind hole 302 containing a solution of magnetic beads to be detected, and the signals are collected through an objective lens 305, and imaged on a camera 311 through a lens 310. If the signal molecule is fluorescent group or particle, the second light source (such as an excitation light source) 306 excites the signal molecule on the microparticles through the lens 307, the dichroic beamsplitter 308 and the objective lens 305, and the generated fluorescent signals are collected through the objective lens 305, transmitted through the dichroic beamsplitter 308, filtered through the filter 309 to remove background signals, and imaged on the camera 311 through the lens 310. The fluorescent signals are collectively referred to herein as dark field imaging. Bright field imaging, which provides information on the number of dispersed microparticles, and dark field imaging, which provides information on the number of the microparticles containing the signal molecule in these dispersed microparticles, are performed alternately; preferably, the concentration of target molecule can be further analyzed by any suitable algorithm, including but not limited to standard curve and Poisson distribution. After the data acquisition is finished, the sample continues to rotate from position 1, via positions 8, 7 and 6, to position 5 (i.e. performing multiple elution to recover the microparticles), during which the blind hole 202 is rinsed to perform the next cycle of detection.

It should be noted that:

(1) when the microparticles are immobilized with agar, after the sample is loaded and moved from position 5 to position 4 for adding agar, the blind hole at position 6 is moved to position 5 to start loading the next sample, and so on, to ensure that each blind hole moves to this position to load a sample to realize a cyclical detection with a high-efficiency;

(2) in addition to immobilizing the microparticles using agar, the microparticles solution 301 with the immunocomplex comprising a signal molecule after the reaction may be pipetted onto a transparent flat solid phase support (quartz, glass, etc.), or a transparent porous solid phase support, and then the microparticles may be plated closely on the solid phase support using a magnet, gravity, or magnetic field directly. Preferably, the solvent of the microparticles solution 301 is removed, and the microparticles are then detected directly by a two-dimensional distribution; and

(3) when the biomolecule is a nucleic acid, it can also be analyzed using the same analysis apparatus with the same apparatus configuration.

**Example 1 Screening of magnetic beads**

Solutions of magnetic beads with different particle sizes (500 nm, 1 $\mu$m, 2 $\mu$m, 3 $\mu$m) were provided. The above solutions of magnetic beads with the four particle sizes were randomly distributed on glass slides, and observed and photographed under a microscope, and the results are shown in FIG. 3.

As can be seen from FIG. 3, when the particle size of the magnetic beads was 500 nm, the magnetic beads had an agglomeration effect and could not be counted individually. The magnetic beads with a particle size larger than 10 $\mu$m were easy to sink due to gravity in an incubation process, and were distributed unevenly in an incubation solution, which affected the collision probability, and reduced the detection sensitivity. The magnetic beads with a particle size of 600 nm to 10 $\mu$m could avoid the two above-mentioned influencing factors. Under different particle sizes and corresponding specific magnifications, the image characteristics presented by bright field microscopic imaging are darkness in the periphery and brightness in the center (the particle size as shown in FIG. 3 is 3 $\mu$m). These characteristics in combination with the sizes of magnetic beads could be used to distinguish magnetic beads from other impurities, so that the detection and analysis results are improved. The method has a high detection tolerance to a contaminated sample and a sample with complex background, such as a pharyngeal swab, saliva, a nasal swab, alveolar lavage fluid and other smear swab samples.

The magnetic beads with a particle size of 1 $\mu$m and 2 $\mu$m shown in FIG. 3 also exhibited the above-mentioned characteristics of darkness in the periphery and brightness in the center (not shown) at their respective specific magnifications.

**Example 2 Detection of different concentrations of Biotin-Qbeads by streptavidin-modified magnetic beads**

1. Experimental Components

Streptavidin-modified magnetic beads, biotin-modified quantum dot beads (Biotin-Qbeads), Buffer A (2% BSA in 10 mM PBS, pH 7.4), Buffer B (0.5% Tween 20 in 10 mM PBS, pH 7.4).

2. Experimental Method

(1) Biotin-Qbeads were diluted to 0, 0.05, 0.25, 0.5, 2.5, 5, 25, 50 fM with Buffer A. Streptavidin-modified beads were diluted to 2 X $10^7$/mL.

(2) 10 $\mu$L of diluted biotin-Qbeads with different concentrations, 10 $\mu$L of diluted streptavidin-modified magnetic beads and 80 $\mu$L of Buffer A were mixed by vortexing, and reacted for 1 h at 37°C.

(3) The supernatant was removed by rinsing six times with Buffer B.

(4) The magnetic beads were resuspended in 20 $\mu$L of PBS, and 5 $\mu$L of suspension was transferred to a coverslip. The magnetic beads were attached to the bottom of the coverslip using a magnet, and single molecule imaging was performed using a fluorescence microscope.

(5) The magnetic beads distributed on the bottom surface of the glass were imaged under a bright field and a fluorescence imaging mode respectively using a low-power objective lens to obtain two sets of data of bright field and the fluorescence. A concentration of antigen could be determined by the ratio of the number of magnetic beads containing the immune complex (i.e., the number of active beads) to the total number of magnetic beads.

(6) Measurements were performed on a series of different concentrations, with each concentration point being repeated three times.

3. Experimental Results

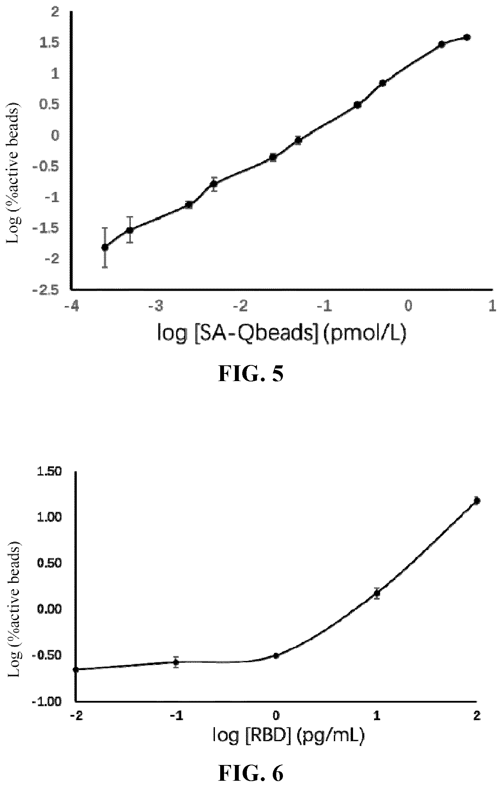

The experimental results are shown in FIG. 5. The detection concentrations from 50 aM to 50 fM are within the linear range of this detection method. Therefore, the linear dynamic range of this method has 3 orders of magnitude. In the example, the limit sensitivity of the system was verified in a reaction system without an antibody, and with the participation of only two reactants with a stronger affinity, streptavidin and biotin, and the results show that if the affinity of the antibody is high enough, the method would be used for detection at a concentration as low as aM, providing a new technical means for detecting low-abundance factors.

**Example 3 Detection of purified Spike protein of novel coronavirus**

1. Experimental Components

Carboxyl-modified magnetic beads, capture antibody (SinoBiological 40150-D006), detection antibody (SinoBiological 40591-MM43), streptavidin-modified quantum dot beads, Spike protein (RBD, SinoBiological), N-hydroxysulfosuccinimide (S-NHS), 1-(3-dimethylaminopropyl)-3-ethylcarbodiimide hydrochloride (EDC), PBS buffer, Buffer A (0.1% Tween 20 in 10 mM PBS, pH 7.4), Buffer B (2% BSA in 10 mM PBS, pH 7.4), Buffer C (0.5% Tween 20 in 10 mM PBS, pH 7.4), MES, Tris-HCl, NaOH, EZ-LinkNHS-PEG 4-Biotinylation Kit, and desalting column (Zeba™ Spin Desalting Columns).

2. Preparation Mthod

2.1 Covalent coupling of magnetic beads with a capture antibody

(1) 20 uL of the magnetic bead suspension was taken into a 1.5 mL EP tube, the EP tube was placed in a magnetic separation rack for enriching the magnetic beads, and the supernatant was removed.
(2) 0.5 mL of $H_2O$ was taken into a centrifuge tube, the magnetic beads were mixed uniformly by vortexing for 15 s, the EP tube was placed in the magnetic separation rack for enriching the magnetic beads, and the supernatant was removed.
(3) 0.5 mL of NaOH was added into the centrifuge tube, the magnetic beads were mixed uniformly by vortexing for 15 s, the EP tube was placed in the magnetic separation rack for enriching the magnetic beads, and the supernatant was removed. This procedure was repeated twice.
(4) 0.5 mL of $H_2O$ was added into the centrifuge tube, the magnetic beads were mixed uniformly by vortexing for 15 s, the EP tube was placed in a magnetic separation rack for enriching the magnetic beads, and the supernatant was removed. This procedure was repeated once.
(5) 0.5 mL of MES (pH 5.0) was added into the centrifuge tube, the magnetic beads were mixed uniformly by vortexing for 15 s, the EP tube was placed in a magnetic separation rack for enriching the magnetic beads, and the supernatant was removed. This procedure was repeated once.
(6) 0.1 mL MES (pH 5.0) was added into the centrifuge tube, the magnetic beads were mixed uniformly by vortexing for 15 s, then 0.1 mL 100 mg/ml EDC and 50 mg/ml S-NHS were added, and the mixture was vortexed for 15 s. The EP tube was placed in a horizontal shaker, and the mixture was reacted for 40 min at room temperature. The EP tube was placed in the magnetic separation rack for enriching magnetic beads, and the supernatant was removed.
(7) 0.5 mL of MES (pH 5.0) was added in the centrifuge tube, the magnetic beads were mixed uniformly by vortexing for 15 s, the EP tube was placed in the magnetic separation rack for enriching the magnetic beads, and the supernatant was removed. This procedure was repeated once. The beads were resuspended by adding 0.1 mL of MES (pH 5.0), and the mixture was vortexed for 15 s.
(8) 88 μg of capture antibody (40150-D006) was diluted with 0.1 mL of MES (pH 5.0) and added to the magnetic bead suspension, and the mixture was vortexed for 15 s. The EP tube was placed in a horizontal shaker, and the mixture was reacted for 1 h at room temperature.
(9) The EP tube was placed in the magnetic separation rack for enriching magnetic beads, and the supernatant was removed. The beads were resuspended by adding 0.4 mL of Tris-HCl (pH 7.4), and the mixture was vortexed for 15 s; the EP tube was placed in the horizontal shaker, and the mixture was reacted for 1 h at room temperature.
(10) The EP tube was placed in the magnetic separation rack for enriching magnetic beads, and the supernatant was removed. 0.5 mL of Buffer A was added, and the mixture was vortexed for 15 s to uniformly mix the magnetic beads; the EP tube was placed in the magnetic separation rack for enriching the magnetic beads, and the supernatant was emoved. This procedure was repeated twice.
(11) 0.5 mL of PBS was added and the mixture was vortexed for 15 s for uniformly mixing the magnetic beads, the EP tube was placed in the magnetic separation rack for enriching the magnetic beads, and the supernatant was removed.
(12) 0.15 mL of Buffer B was added, the mixture was vortexed for 15 s for uniformly mixing the beads and stored at 4°C.

2.2 Biotinylation modification of detection antibody

(1) 1 mg of detection antibody was diluted with 1 mL of 10 mM PBS to a concentration of 1 mg/mL, and was stored at 4°C for later use.
(2) One tube of NHS-PEG4-Biotin packed in the kit was dissolved by adding 0.17 mL of ultrapure water to obtain an NHS-PEG 4-Biotin solution with a concentration of 20 μM.

(3) 6.65μL of NHS-PEG4-Biotin solution was added to the detection antibody solution, and the mixture was reacted for 1 h at room temperature.

(4) Buffer was displaced using a desalting column (Zeba™ Spin Desalting Columns), and excess NHS-PEG 4-Biotin solution was removed from the system at the same time.

(5) The concentration of biotinylated detection antibody was determined using Nanodrop, and the mixtuer was stored at 4°C.

3. Experimental Method

(1) The Spike protein was diluted to concentrations of 0, 0.01, 0.1, 1, 10 and 100 pg/mL with Buffer B.

(2) The magnetic beads labeled with the capture antibody were diluted 50-fold with Buffer B, the biotin-labeled detection antibody was diluted to a concentration of 4 μg/mL, and SA-Qbeads was diluted to 1.67 nM.

(3) 25 μL of diluted Spike proteins with different concentrations, the magnetic beads labeled with the capture antibody, biotin labeled detection antibody and SA-Qbeads were taken respectively, uniformly mixed by vortexing, and reacted for 1 h at 37°C.

(4) The mixture was rinsed six times with Buffer C, and the supernatant was removed.

(5) The beads were resuspended by adding 20 μL of PBS, and 5 μL of suspension was transferred to a coverslip. The beads were attached to the bottom of the coverslip using a magnet, and single molecule imaging was performed using a fluorescence microscope.

(6) The magnetic beads distributed on the bottom surface of the glass were imaged under a bright field and a fluorescence imaging mode respectively by using a low-power objective lens, to obtain two sets of data of bright field and fluorescence. A concentration of antigen could be determined by the ratio of the number of magnetic beads containing the immune complex (i.e., the number of active beads) to the total number of magnetic beads.

(7) Measurements were performed on a series of Spike protein concentrations, with each concentration point being repeated three times.

4. Experimental Results

The detection results are shown in FIG. 6, and it can be seen that in this example, the detection range for Spike protein was 0.01 pg/mL to 100 pg/mL, and the lower limit of detection could reach 10 fg/mL.

**Comparative Example 1: Detection of pseudovirus expressing novel coronavirus S protein on surface**

1. Experimental Components

The detection sample was a pseudovirus expressing novel coronavirus S protein on the surface, and the remaining experimental components were identical to those in Example 3.

2. Preparation method:

2.1 Covalent coupling of magnetic beads with capture antibody

The procedure was conducted identically to the corresponding procedure in Example 3.

2.2 Biotinylation modification of detection antibody

The procedure was conducted identically to the corresponding procedure in Example 3.

3. Experimental Method

Pseudovirus was used as the detection sample, including solutions without the pseudovirus and a series of solutions containing 2, 5, 20, 100 and 200 pseudoviruses per 100 μL. The remaining steps were performed identically to the corresponding steps in Example 3.

4. Experimental Results

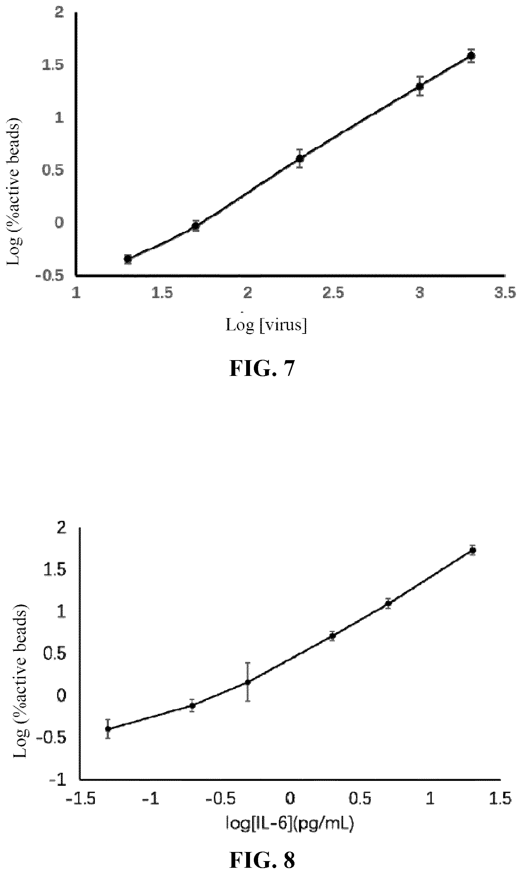

It can be seen from the results in FIG. 7 that the number of pseudovirus detected from 2 to 200 is within the linear range of the detection method. In the case that the number of pseudovirus is 2 or 5, although the ratio of the number of fluorescent magnetic beads to the total number of magnetic beads is lower, it can still be significantly distinguished from the control group without the virus. The current detection sensitivity of the same antibody in the chemiluminescence platform is about 50 pseudoviruses, and the result of the example is 25 times higher than that of the chemiluminescence method.

**Example 4 Magnetic bead assay for detection of IL-6**

1. Experimental Components

In the experiment, the capture antibody is 8C9 (Cnpair Biotech Co., Ltd.), the detection antibody is 9A2 (Cnpair Biotech Co., Ltd.), the detection sample is IL-6, and the other experimental components were identical to those in Example 1.

## 2. Preparation Method

### 2.1 Covalent coupling of magnetic beads with capture antibody

The procedure was conducted identically to the corresponding procedure in Example 1.

### 2.2 Biotinylation modification of detection antibody

The procedure was conducted identically to the corresponding procedure in Example 1.

## 3. Experimental Method

(1) IL-6 was diluted to concentrations of 0, 0.05, 0.2, 0.5, 2, 5 and 20 pg/mL with Buffer B.
(2) The magnetic beads labeled with capture antibody were diluted 150-fold with Buffer B, the biotin-labeled detection antibody was diluted to a concentration of 4 $\mu$g/mL, and SA-Qbeads were diluted to 8 nM. The remaining procedures were performed identically to the corresponding procedures in Example 1.

## 4. Experimental Results

The detection results are shown in FIG. 8, it can be seen that in this example, the detection range of IL-6 is from 0.05 pg/mL to 20 pg/mL, which is within the linear range of the detection method, and the lower limit of detection could reach 50 fg/mL. The current detection sensitivity of the same antibody is higher than 1 pg/ml in a chemiluminescence platform, and the result of the example is about 20 times higher than that of a chemiluminescence method.

## Claims

1. A method for detecting a signal molecule, **characterized in that**, the method comprises the following steps of:

(1) providing a solution comprising microparticles, wherein the microparticles comprise microparticles binding to the signal molecule to be detected;
(2) immobilizing the microparticles in the solution to the surface and/or the interior of a solid phase support;
(3) counting the microparticles in a selected field of view under a bright field; and
(4) counting the microparticles binding to the signal molecule in a selected field of view under a dark field; and

(5) determining the concentration of the signal molecule according to the counting results obtained in steps (3) and (4);
wherein step (3) comprises removing the agglomerated/overlapped microparticles in the bright field, and wherein the selected field of view under the bright field and the selected field of view under the dark field are the same field of view, and counting under the bright and dark fields are performed in the same field of view.

2. A method for detecting one or more target molecules, **characterized in that**, the method comprises the following steps of:

(1) providing a solution comprising microparticles, wherein a target molecule forms a complex by a specific binding reaction, the microparticles is linked to the complex, and the complex is labeled with a signal molecule;
(2) immobilizing the microparticles in the solution to the surface and/or the interior of a solid phase support;
(3) counting the microparticles in a selected field of view under a bright field;
(4) counting the microparticles binding to the complex in a selected field of view under a dark field; and
(5) determining the concentration of the signal molecule according to the counting results obtained in steps (3) and (4), and further determining the concentration of the target molecule;
wherein step (3) comprises removing the agglomerated/overlapped microparticles in the bright field, and wherein the selected field of view under the bright field and the selected field of view under the dark field are the same field of view, and counting under the bright and dark fields are performed in the same field of view.

3. The method according to claim 1 or 2, **characterized in that**, in step (2), the solid phase support does not need to be spatially divided to achieve a random distribution, wherein the solid phase support is preferably selected from a multi-well plate, a flat plate or a flow channel, and/or
wherein in step (2), the microparticles are immobilized to the surface and/or the interior of the solid phase support by an applied magnetic field and/or an electric field and/or a gel.

4. The method according to claim 1 or 2, **characterized in that**, an uncertain quantity of microparticles are immobilized to the surface and/or the interior of the solid support.

5. The method according to claim 2, **characterized in that**, the target molecule is one or more selected from the group consisting of a protein, a polypeptide, an amino acid, an antigen, a receptor, a ligand, and a nucleic acid, wherein the target molecule is preferably an antibody, and/or
wherein the specific binding reaction is one or more selected from the group consisting of an immune reaction, a hybridization reaction, and a receptor-ligand interaction.

6. The method according to claim 1 or 2, **characterized in that**, the microparticles are magnetic microparticles, preferably magnetic beads, wherein the magnetic beads preferably have a particle size ranging from 600 nm to 10 $\mu$m, in particular from 1 $\mu$m to 5 $\mu$m.

7. The method according to claim 1 or 2, **characterized in that**, the signal molecule is one or more selected from the group consisting of a chromophore, a digoxin-labeled probe, a metal nanoparticle, and an enzyme or form the group consisting of an organic small molecule fluorescent probe, a quantum dot, a fluorescent bead, a three-dimensional DNA nanostructure reporter probe, an upconversion luminescent nanomaterial bead, a rolling circle amplification fluorescent molecule amplification structure, and a nucleic acid aptamer fluorescent molecule amplification structure, wherein the signal molecule is preferably a quantum dot bead, which preferably has a size within 110 nm.

8. The method according to claim 1 or 2, **characterized in that**, the coordinates of the microparticles in the image are determined by bright field microscopic imaging,

wherein the coordinates of the microparticles are preferably determined from the difference in brightness of the microparticles, the difference in brightness of the microparticles preferably is the brightness difference of the microparticles themselves,
wherein the counting in step (4) is preferably determined by the coordinates of the microparticles in the image.

9. The method according to claim 1 or 2, **characterized in that**, the method for determining the concentration of the target molecule in step (5) comprises:
determining the concentration of the target molecule according to a proportional relation between the numbers of the microparticles obtained in step (3) and step (4) in combination with a standard curve.

10. Use of the method according to any one of claims 1 to 9 in the preparation of a diagnostic reagent for detecting a biomolecule.

11. Use of a detection apparatus for implementing the method according to any one of claims 1 to 9, comprising:

a solid phase support (201) capable of immobilizing microparticles, the microparticles comprising microparticles binding to a signal molecule to be detected and being dispersed at the surface and/or in the interior of the solid phase support (201);
at least one first light source irradiating microparticles within a selected field of view of the solid phase support (201) to form bright field signals related to the total number of microparticles, and at least one second light source irradiating microparticles within a selected field of view of the solid phase support (201) to form dark field signals related to the total number of microparticles binding to the signal molecule to be detected;
a signal acquisition unit for acquiring the bright field signals and dark field signals; and
a signal processing unit for determining the concentration of the signal molecule according to the acquired bright field signals and dark field signals,
the selected field of view for the at least one first light source and the selected field of view for the at least one second light source are the same field of view.

12. The use according to claim 11, **characterized in that**, the signal acquisition unit comprises an amplification assembly for amplifying microparticles within the selected field of view, the amplification assembly is preferably an objective lens (305), in particular a low-power objective lens, and/or
the signal acquisition unit comprises at least one photographing assembly (311).

13. The use according to claim 11, **characterized in that**, the solid phase support (201) is at least a partially optically transparent support, wherein the solid phase support (201) is preferably disposed above or below the signal acquisition unit, preferably removable from above or below the signal acquisition unit, and/or

wherein the solid phase support (201) comprises at least one flow channel comprising an inlet and an outlet, and a solution comprising the microparticles is dispersed within the flow channel, and/or
wherein the solid phase support (201) is a multi-well plate or a flat plate.

14. The use according to claim 11, **characterized in that**, the apparatus further comprises a magnetic field generating device or an electric field generating device for immobilizing and dispersing the microparticles at the surface and/or in the interior of the solid phase support (201), and/or

the apparatus further comprises a displacement mechanism for actuating the signal acquisition unit/solid phase support, wherein the displacement mechanism preferably is one or more selected from the group consisting of a one-dimensional displacement stage, a two-dimensional displacement stage, and a three-dimensional displacement stage.

15. The use according to claim 11, **characterized in that**, the solid phase support (201) is a turntable which can rotate relative to the signal acquisition unit, wherein the turntable (201) comprises at least one optically transparent detection site which is detected by the signal acquisition unit when the detection site is located in an optical path of the signal acquisition unit, wherein the turntable (201) is preferably configured to rotate sequentially between a plurality of stations in a stepwise manner and to perform the following operations on a solution to be detected at the detection site at the plurality of stations: immobilizing and rinsing microparticles in the solution,

wherein the plurality of stations preferably comprise a detection station located in an optical path of the signal acquisition unit, at least one pre-treatment station located upstream of the detection station, and at least one post-treatment station located downstream of the detection station, wherein an operation of immobilizing and dispersing microparticles in the solution is performed at the pre-treatment station, and a rinsing operation is performed at the post-treatment station.

**Patentansprüche**

1. Verfahren zum Detektieren eines Signalmoleküls, **dadurch gekennzeichnet, dass** das Verfahren die folgenden Schritte umfasst:

   (1) Bereitstellen einer Lösung umfassend Mikropartikel, wobei die Mikropartikel Mikropartikel umfassen, die das zu detektierende Signalmolekül binden;
   (2) Immobilisieren der Mikropartikel in der Lösung an der Oberfläche und/oder im Inneren eines Festphasenträgers;
   (3) Zählen der Mikropartikel in einem ausgewählten Sichtfeld unter einem Hellfeld; und
   (4) Zählen der an das Signalmolekül bindenden Mikropartikel in einem ausgewählten Sichtfeld unter einem Dunkelfeld; und
   (5) Bestimmen der Konzentration des Signalmoleküls gemäß der in Schritten (3) und (4) erhaltenen Zählergebnisse;
wobei Schritt (3) das Entfernen der agglomerierten/überlappten Mikropartikel im Hellfeld umfasst, und wobei das ausgewählte Sichtfeld unter dem Hellfeld und das ausgewählte Sichtfeld unter dem Dunkelfeld dasselbe Sichtfeld sind, und das Zählen unter dem Hell- und dem Dunkelfeld im selben Sichtfeld durchgeführt wird.

2. Verfahren zum Detektieren eines oder mehrerer Zielmoleküle, **dadurch gekennzeichnet, dass** das Verfahren die folgenden Schritte umfasst:

   (1) Bereitstellen einer Lösung umfassend Mikropartikel, wobei ein Zielmolekül durch eine spezifische Bindungsreaktion einen Komplex bildet, die Mikropartikel mit dem Komplex verknüpft sind, und der Komplex mit einem Signalmolekül markiert ist;
   (2) Immobilisieren der Mikropartikel in der Lösung an der Oberfläche und/oder im Inneren eines Festphasenträgers;
   (3) Zählen der Mikropartikel in einem ausgewählten Sichtfeld unter einem Hellfeld;
   (4) Zählen der an den Komplex bindenden Mikropartikel in einem ausgewählten Sichtfeld unter einem Dunkelfeld; und
   (5) Bestimmen der Konzentration des Signalmoleküls gemäß der in Schritten (3) und (4) erhaltenen Zählergebnisse, und ferner Bestimmen der Konzentration des Zielmoleküls,
   wobei Schritt (3) das Entfernen der agglomerierten/überlappten Mikropartikel im Hellfeld umfasst, und wobei das ausgewählte Sichtfeld unter dem Hellfeld und das ausgewählte Sichtfeld unter dem Dunkelfeld dasselbe Sichtfeld sind, und das Zählen unter dem Hell- und Dunkelfeld im selben Sichtfeld durchgeführt wird.

3. Verfahren nach Anspruch 1 oder 2, **dadurch gekennzeichnet, dass** in Schritt (2) der Festphasenträger nicht räumlich aufgeteilt werden muss, um eine zufällige Verteilung zu erreichen, wobei der Festphasenträger vorzugsweise ausgewählt ist aus einer Multiwellplatte, einer flachen Platte oder einem Strömungskanal, und/oder
wobei in Schritt (2) die Mikropartikel durch ein angelegtes Magnetfeld und/oder ein elektrisches Feld und/oder ein Gel an der Oberfläche und/oder im Inneren des Festphasenträgers immobilisiert werden.

4. Verfahren nach Anspruch 1 oder 2, **dadurch gekennzeichnet, dass** eine unbestimmte Menge an Mikropartikeln an der Oberfläche und/oder im Inneren des festen Trägers immobilisiert sind.

**5.** Verfahren nach Anspruch 2, **dadurch gekennzeichnet, dass** das Zielmolekül ein oder mehrere aus der Gruppe, bestehend aus einem Protein, einem Polypeptid, einer Aminosäure, einem Antigen, einem Rezeptor, einem Liganden und einer Nukleinsäure, ist, wobei das Zielmolekül vorzugsweise ein Antikörper ist, und/oder
wobei die spezifische Bindungsreaktion eine oder mehrere ausgewählt aus der Gruppe, bestehend aus einer Immunreaktion, einer Hybridisierungsreaktion und einer Rezeptor-Liganden-Interaktion, ist.

**6.** Verfahren nach Anspruch 1 oder 2, **dadurch gekennzeichnet, dass** die Mikropartikel magnetische Mikropartikel sind, vorzugsweise magnetische Beads, wobei die magnetischen Beads vorzugsweise eine Partikelgröße im Bereich von 600 nm bis 10 µm, insbesondere von 1 µm bis 5 µm, aufweisen.

**7.** Verfahren nach Anspruch 1 oder 2, **dadurch gekennzeichnet, dass** das Signalmolekül eines oder mehrere ausgewählt aus der Gruppe, bestehend aus einem Chromophor, einer Digoxin-markierten Sonde, einem Metallnanopartikel und einem Enzym, oder aus der Gruppe, bestehend aus einer organischen niedermolekularen Fluoreszenzsonde, einem Quantenpunkt, einem Fluoreszenzbead, einer dreidimensionalen DNA-Nanostruktur-Reportersonde, einem Upconversion-Lumineszenz-Nanomaterialbead, einer Rolling-Circle-Amplification-Fluoreszenzmolekül-Amplifikationsstruktur und einer Nukleinsäure-Aptamer-Fluoreszenzmolekül-Amplifikationsstruktur, wobei das Signalmolekül vorzugsweise ein Quantenpunktbead ist, der vorzugsweise eine Größe innerhalb von 110 nm aufweist.

**8.** Verfahren nach Anspruch 1 oder 2, **dadurch gekennzeichnet, dass** die Koordinaten der Mikropartikel im Bild durch Hellfeldmikroskopiebildgebung bestimmt werden,

wobei die Koordinaten der Mikropartikel vorzugsweise aus dem Helligkeitsunterschied der Mikropartikel bestimmt werden, wobei der Helligkeitsunterschied der Mikropartikel vorzugsweise der Helligkeitsunterschied der Mikropartikel selbst ist,
wobei das Zählen in Schritt (4) vorzugsweise durch die Koordinaten der Mikropartikel im Bild bestimmt wird.

**9.** Verfahren nach Anspruch 1 oder 2, **dadurch gekennzeichnet, dass** das Verfahren zum Bestimmen der Konzentration des Zielmoleküls in Schritt (5) umfasst:
Bestimmen der Konzentration des Zielmoleküls gemäß einer proportionalen Beziehung zwischen den Anzahlen der in Schritt (3) und Schritt (4) erhaltenen Mikropartikel in Kombination mit einer Standardkurve.

**10.** Verwendung des Verfahrens nach einem der Ansprüche 1 bis 9 bei der Herstellung eines diagnostischen Reagenzes zum Nachweis eines Biomoleküls.

**11.** Verwendung einer Detektionsvorrichtung zum Implementieren des Verfahrens nach einem der Ansprüche 1 bis 9, umfassend:

einen Festphasenträger (201), der in der Lage ist, Mikropartikel zu immobilisieren, wobei die Mikropartikel Mikropartikel umfassen, die an ein zu detektierendes Signalmolekül binden und an der Oberfläche und/oder im Inneren des Festphasenträgers (201) dispergiert sind;
mindestens eine erste Lichtquelle, die Mikropartikel innerhalb eines ausgewählten Sichtfelds des Festphasenträgers (201) bestrahlt, um Hellfeldsignale zu bilden, die mit der Gesamtanzahl der Mikropartikel in Beziehung stehen, und mindestens eine zweite Lichtquelle, die Mikropartikel innerhalb eines ausgewählten Sichtfelds des Festphasenträgers (201) bestrahlt, um Dunkelfeldsignale zu bilden, die mit der Gesamtzahl der Mikropartikel in Beziehung stehen, die das zu detektierende Signalmolekül binden;
eine Signalerfassungseinheit zum Erfassen der Hellfeldsignale und Dunkelfeldsignale; und
eine Signalverarbeitungseinheit zum Bestimmen der Konzentration des Signalmoleküls gemäß der erfassten Hellfeldsignale und Dunkelfeldsignale,
das ausgewählte Sichtfeld für die mindestens eine erste Lichtquelle und das ausgewählte Sichtfeld für die mindestens eine zweite Lichtquelle sind dasselbe Sichtfeld.

**12.** Verwendung nach Anspruch 11, **dadurch gekennzeichnet, dass** die Signalerfassungseinheit eine Verstärkungsanordnung zum Verstärken von Mikropartikeln innerhalb des ausgewählten Sichtfelds umfasst, wobei die Verstärkungsanordnung vorzugsweise eine Objektivlinse (305), insbesondere eine Objektivlinse mit geringer Vergrößerungsleistung, ist, und/oder
die Signalerfassungseinheit mindestens eine Fotografieranordnung (311) umfasst.

**13.** Verwendung nach Anspruch 11, **dadurch gekennzeichnet, dass** der Festphasenträger (201) ein zumindest teilweise optisch transparenter Träger ist, wobei der Festphasenträger (201) vorzugsweise über oder unter der Signalerfassungseinheit angeordnet ist, vorzugsweise von über oder unter der Signalerfassungseinheit entfernbar ist, und/oder

wobei der Festphasenträger (201) mindestens einen Strömungskanal umfasst, der einen Einlass und einen Auslass umfasst, und eine die Mikropartikel umfassende Lösung innerhalb des Strömungskanals dispergiert ist, und/oder wobei der Festphasenträger (201) eine Multiwellplatte oder eine flache Platte ist.

14. Verwendung nach Anspruch 11, **dadurch gekennzeichnet, dass** die Vorrichtung ferner eine Einheit zum Erzeugen eines magnetischen Felds oder eine Einheit zum Erzeugen eines elektrischen Felds zum Immobilisieren und Dispergieren der Mikropartikel an der Oberfläche und/oder im Inneren des Festphasenträgers (201) umfasst, und/oder die Vorrichtung ferner einen Verschiebemechanismus zum Betätigen der Signalerfassungseinheit/- Festphasenträger umfasst, wobei der Verschiebemechanismus vorzugsweise einer oder mehrere ausgewählt aus der Gruppe bestehend aus einer eindimensionalen Verschiebestufe, einer zweidimensionalen Verschiebestufe und einer dreidimensionalen Verschiebestufe ist.

15. Verwendung nach Anspruch 11, **dadurch gekennzeichnet, dass** der Festphasenträger (201) eine Drehscheibe ist, die sich relativ zur Signalerfassungseinheit drehen kann, wobei die Drehscheibe (201) mindestens eine optisch transparente Detektionsstelle umfasst, die von der Signalerfassungseinheit erkannt wird, wenn sich die Detektionsstelle in einem Strahlengang der Signalerfassungseinheit befindet, wobei die Drehscheibe (201) vorzugsweise konfiguriert ist, um sich schrittweise sequentiell zwischen einer Vielzahl von Stationen zu drehen und an einer an der Detektionsstelle zu detektierenden Lösung an der Vielzahl von Stationen die folgenden Vorgänge durchzuführen: Immobilisieren und Spülen von Mikropartikeln in der Lösung, wobei die Vielzahl von Stationen vorzugsweise eine in einem Strahlengang der Signalerfassungseinheit angeordnete Detektionsstation, mindestens eine stromaufwärts der Detektionsstation angeordnete Vorbehandlungsstation und mindestens eine stromabwärts der Detektionsstation angeordnete Nachbehandlungsstation umfasst, wobei an der Vorbehandlungsstation ein Vorgang des Immobilisierens und Dispergierens von Mikropartikeln in der Lösung durchgeführt wird und an der Nachbehandlungsstation ein Spülvorgang durchgeführt wird.

**Revendications**

1. Procédé permettant de détecter une molécule signal,
   **caractérisé en ce que,** le procédé comprend les étapes suivantes consistant à :

   (1) fournir une solution comprenant des microparticules, dans lequel les microparticules comprennent des microparticules se liant à la molécule signal à détecter ;
   (2) immobiliser les microparticules présentes dans la solution sur la surface et/ou à l'intérieur d'un support en phase solide ;
   (3) compter les microparticules dans un champ d'observation choisi sous un champ clair ; et
   (4) compter les microparticules se liant à la molécule signal dans un champ d'observation choisi sous un champ sombre ; et
   (5) déterminer la concentration de la molécule signal selon les résultats de comptage obtenus aux étapes (3) et (4) ;
   dans lequel l'étape (3) comprend l'élimination des microparticules agglomérées/se chevauchant dans le champ clair, et dans lequel le champ d'observation choisi sous le champ clair et le champ d'observation choisi sous le champ sombre sont le même champ d'observation, et les comptages sous les champs clair et sombre sont effectués dans le même champ d'observation.

2. Procédé permettant de détecter une ou plusieurs molécules cibles, **caractérisé en ce que,** le procédé comprend les étapes suivantes consistant à :

   (1) fournir une solution comprenant des microparticules, dans lequel une molécule cible forme un complexe par une réaction de liaison spécifique, les microparticules sont liées au complexe, et le complexe est marqué avec une molécule signal ;
   (2) immobiliser les microparticules présentes dans la solution sur la surface et/ou à l'intérieur d'un support en phase solide ;
   (3) compter les microparticules dans un champ d'observation choisi sous un champ clair ;
   (4) compter les microparticules se liant au complexe dans un champ d'observation choisi sous un champ sombre ; et
   (5) déterminer la concentration de la molécule signal selon les résultats de comptage obtenus aux étapes (3) et (4), et en outre déterminer la concentration de la molécule cible ;
   dans lequel l'étape (3) comprend l'élimination des microparticules agglomérées/se chevauchant dans le champ clair, et dans lequel le champ d'observation choisi sous le champ clair et le champ d'observation choisi sous le champ sombre sont le même champ d'observation, et les comptages sous les champs clair et sombre sont effectués dans le même champ d'observation.

3. Procédé selon la revendication 1 ou 2, **caractérisé en ce que,** à l'étape (2), le support en phase solide n'a pas besoin d'être spatialement divisé pour réaliser une distribution aléatoire, dans lequel le support en phase solide est de préférence choisi parmi une plaque multipuits, une plaque plate ou un canal d'écoulement, et/ou

   dans lequel à l'étape (2), les microparticules sont immobilisées sur la surface et/ou à l'intérieur du support en phase solide par un champ magnétique appliqué et/ou un champ électrique et/ou un gel.

4. Procédé selon la revendication 1 ou 2, **caractérisé en ce que,** une quantité incertaine de microparticules sont immobilisées sur la surface et/ou à l'intérieur du support solide.

5. Procédé selon la revendication 2, **caractérisé en ce que,** la molécule cible est une ou plusieurs choisies dans le groupe constitué de protéine, polypeptide, acide aminé, antigène, récepteur, ligand, et acide nucléique, dans lequel la molécule cible est de préférence un anticorps, et/ou

   dans lequel la réaction de liaison spécifique est une ou plusieurs choisies dans le groupe constitué de réaction immunitaire, réaction d'hybridation, et interaction récepteur-ligand.

6. Procédé selon la revendication 1 ou 2, **caractérisé en ce que,** les microparticules sont des microparticules magnétiques, de préférence des billes magnétiques, dans lequel les billes magnétiques sont de préférence une taille de particule allant de 600 nm à 10 $\mu$m, en particulier de 1 $\mu$m à 5 $\mu$m.

7. Procédé selon la revendication 1 ou 2, **caractérisé en ce que,** la molécule signal est une ou plusieurs choisies dans le groupe constitué de chromophore, sonde marquée par la digoxine, nanoparticule métallique, et enzyme ou dans le groupe constitué de sonde fluorescente à petite molécule organique, point quantique, bille fluorescente, sonde rapporteur à nanostructure d'ADN tridimensionnelle, bille de nanomatériau luminescent à conversion ascendante, structure d'amplification de molécule fluorescente d'amplification en cercle roulant, et structure d'amplification de molécule d'aptamère d'acide nucléique fluorescent, dans lequel la molécule signal est de préférence une bille de points quantiques, qui de préférence a une taille de moins de 110 nm.

8. Procédé selon la revendication 1 ou 2, **caractérisé en ce que,** les coordonnées des microparticules dans l'image sont déterminées par imagerie microscopique en champ clair,

dans lequel les coordonnées des microparticules sont de préférence déterminées à partir de la différence de clarté des microparticules, la différence de clarté des microparticules est de préférence la différence de clarté des microparticules elles-mêmes,

dans lequel le comptage à l'étape (4) est de préférence déterminé par les coordonnées des microparticules dans l'image.

9. Procédé selon la revendication 1 ou 2, **caractérisé en ce que,** le procédé permettant de déterminer la concentration de la molécule cible à l'étape (5) comprend :

   la détermination de la concentration de la molécule cible selon une relation proportionnelle entre les nombres des microparticules obtenus à l'étape (3) et à l'étape (4) en combinaison avec une courbe d'étalonnage.

10. Utilisation du procédé selon l'une quelconque des revendications 1 à 9 dans la préparation d'un réactif de diagnostic permettant de détecter une biomolécule.

11. Utilisation d'un appareil de détection permettant de mettre en œuvre le procédé selon l'une quelconque des revendications 1 à 9, comprenant :

   un support en phase solide (201) capable d'immobiliser des microparticules, les microparticules comprenant des microparticules se liant à une molécule signal à détecter et étant dispersées au niveau de la surface et/ou dans l'intérieur du support en phase solide (201) ;
   au moins une première source de lumière éclairant les microparticules au sein d'un champ d'observation choisi du support en phase solide (201) pour former des signaux en champ clair se rapportant au nombre total de microparticules, et au moins une seconde source de lumière éclairant les microparticules au sein d'un champ d'observation choisi du support en phase solide (201) pour former des signaux en champ sombre se rapportant au nombre total de microparticules se liant à la molécule signal à détecter ;
   une unité d'acquisition de signaux permettant d'acquérir les signaux en champ clair et les signaux en champ sombre ; et
   une unité de traitement de signaux permettant de déterminer la concentration de la molécule signal selon les signaux en champ clair et les signaux en champ sombre acquis,
   le champ d'observation choisi pour l'au moins une première source de lumière et le champ d'observation choisi pour l'au moins une seconde source de lumière étant le même champ d'observation.

**12.** Utilisation selon la revendication 11, **caractérisée en ce que,** l'unité d'acquisition de signaux comprend un ensemble d'amplification permettant d'amplifier des microparticules au sein du champ d'observation choisi, l'ensemble d'amplification étant de préférence une lentille d'objectif (305), en particulier une lentille d'objectif de faible puissance, et/ou l'unité d'acquisition de signaux comprend au moins un ensemble de photographie (311).

**13.** Utilisation selon la revendication 11, **caractérisée en ce que,** le support en phase solide (201) est au moins un support partiellement optiquement transparent, dans laquelle le support en phase solide (201) est de préférence disposé au-dessus ou au-dessous de l'unité d'acquisition de signaux, de préférence amovible du dessus ou du dessous de l'unité d'acquisition de signaux, et/ou

dans laquelle le support en phase solide (201) comprend au moins un canal d'écoulement comprenant une entrée et une sortie, et une solution comprenant les microparticules est dispersée au sein du canal d'écoulement, et/ou dans lequel le support en phase solide (201) est une plaque multipuits ou une plaque plate.

**14.** Utilisation selon la revendication 11, **caractérisée en ce que,** l'appareil comprend en outre un dispositif de génération de champ magnétique ou un dispositif de génération de champ électrique permettant d'immobiliser et de disperser les microparticules au niveau de la surface et/ou dans l'intérieur du support en phase solide (201), et/ou l'appareil comprend en outre un mécanisme de déplacement permettant d'actionner l'unité d'acquisition de signaux/le support en phase solide, dans laquelle le mécanisme de déplacement est de préférence un ou plusieurs choisis dans le groupe constitué de platine de déplacement unidimensionnel, platine de déplacement bidimensionnel, et platine de déplacement tridimensionnel.

**15.** Utilisation selon la revendication 11, **caractérisée en ce que,** le support en phase solide (201) est un plateau tournant qui peut tourner par rapport à l'unité d'acquisition de signaux, dans laquelle le plateau tournant (201) comprend au moins un site de détection optiquement transparent qui est détecté par l'unité d'acquisition de signaux lorsque le site de détection est situé dans un trajet optique de l'unité d'acquisition de signaux, dans laquelle le plateau tournant (201) est de préférence conçu pour tourner séquentiellement par étapes entre une pluralité de stations et pour effectuer les opérations suivantes sur une solution à détecter au niveau du site de détection au niveau de la pluralité de stations : l'immobilisation et le rinçage de microparticules présentes dans la solution,

dans laquelle la pluralité de stations comprend de préférence une station de détection située dans un trajet optique de l'unité d'acquisition de signaux, au moins une station de prétraitement située en amont de la station de détection, et au moins une station de post-traitement située en aval de la station de détection, dans laquelle une opération d'immobilisation et de dispersion de microparticules dans la solution est effectuée au niveau de la station de prétraitement, et une opération de rinçage est effectuée au niveau de la station de post-traitement.

| Magnetic beads coated with capture antibodies | Antigens to be detected | Detection antibodies binding to reporters | Forming IC adhered to magnetic beads |

**FIG. 1**

Droplets containing magnetic beads and IC

Microscopic imaging of magnetic beads under bright field

**FIG. 2**

FIG. 3

FIG. 4

**FIG. 5**

**FIG. 6**

**FIG. 7**

**FIG. 8**

**FIG. 9**

**FIG. 10**

**FIG. 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 12731130 B
- CN 201280049085
- CN 20208000774
- WO 2019068269 A1, Koole

**Non-patent literature cited in the description**

- *LAB ON A CHIP*, vol. 20 (1), 2113-2121
- **MOK J** ; **MINDRINOS MN** ; **DAVIS RW** ; **JAVANMARD M**. Digital microfluidic assay for protein detection. *PROCEEDINGS OF THE NATIONAL ACADEMY OF SCIENCES*, 2014, vol. 111 (6), 2110-2115
- **KOOLE R** ; **VAN SCHOONEVELD MM** ; **HILHORST J** ; **CASTERMANS K** ; **CORMODE DP** ; **STRIJKERS GJ** ; **DE MELLO DONEGÁ C** ; **VANMAEKELBERGH D** ; **GRIFFIOEN AW** ; **NICOLAY K**. Paramagnetic Lipid-Coated Silica Nanoparticles with a Fluorescent Quantum Dot Core: A New Contrast Agent Platform for Multimodality Imaging.. *BIOCONJUGATE CHEMISTRY*, 2008, vol. 19 (12), 2471-2479